# EUROPEAN PATENT APPLICATION

(11) **EP 2 071 481 A2**
(43) Date of publication of application: **17.06.2009**
(21) Application number: 09153661.5
(22) Date of filing: 14.09.2001
(51) Int. Cl.: G06F 19/00, C40B 30/04, G01N 33/68

(54) **Method system, apparatus and device for discovering and preparing chemical compounds for medical and other uses**

(30) Priority: 14.09.2000 US 232626 P; 12.01.2001 US 260867 P; 05.03.2001 US 272774 P; 01.06.2001 US 294563 P; 19.06.2001 US 298900 P
(62) Divisional of application: 01965662.8
(71) Applicant: Reverse Proteomics Research Institute Co., Ltd, Chiyoda-ku Tokyo 101-0044 (JP)
(72) Inventor: ISOGAI, Takao, Ibaraki 300-0303 (JP); HIRAYAMA, Noriaki, Kanagawa 228-0023 (JP); NAGASHIMA, Renpei, Tokyo 170-0002 (JP)
(74) Representative: Vossius & Partner

(57) **Abstract**

Disclosed in this invention are methods, systems, databases, user-interfaces, software, media, and services useful for evaluating interactions between chemical compounds and proteins and for utilizing the information resulting from such evaluation for the purpose of discovering chemical compounds for medical and other fields. An approach termed "reverse proteomics" is disclosed. This invention generates an enormously large pool of new target proteins for drug discovery, novel methods for designing of new drugs, and a previously unthinkable pool of virtually synthesized small molecules for therapeutic uses. This invention is also applicable, for example, to discovery of substitutes for environmentally hazardous chemicals, more effective agrochemicals, and healthier food additives.

## Description

### Technology Fields

This invention relates to the method, system, apparatus and device for discovering and preparing chemical compounds for medical and other uses. Other uses include but not limited to those in agrochemical, food, environmental, fermentation, and veterinary fields.

### Background Technology

Research for discovery and development of new drugs begins with exploration, identification, characterization, and validation of drug targets. Hereafter in this specification the phrase "identification of target" is to mean identification of characterized target.

Currently popular steps of drug discovery research are to study the genome of humans and other organisms, identify certain genes (the job of genomics) which upon transcription and translation produce proteins, characterize the function of proteins (the job of proteomics), and, if proteins are thought to be likely drug targets, screen a large number of chemical compounds for their activity to modulate the function of proteins. Recent development in genomics along with that in proteomics is hoped to accelerate identification of such drug targets and ultimately lead to the discovery of new drugs that satisfy unmet medical needs. This can be called one-way upstream-to-downstream genomics/proteomics approach. However, while the DNA sequence of more than 90% of human genome has become known, most of the genes that are embedded in the genome are yet to be identified, the function of proteins that are encoded by genes are to be elucidated, and the interactions among proteins are to be characterized. As to other mammals than humans our knowledge of their genome is scarce. Proteomics is still in its embryonic stage of development. At present, therefore, it is difficult to state that we have reached a stage where we are able to effectively identify likely drug targets through the one-way genomic/proteomic approach.

Another common approach is to select a drug target protein, frequently abbreviated in this specification to target protein or drug target, once the function of the protein has become known through research other than the one-way genomic/proteomic approach illustrated above. Enzymes and ion channels, cell surface receptors of neurotransmitters and cytokines, and nuclear receptors of steroids, retinoic acids and vitamin D3 are such examples. Proteins associated with signal transduction, notably kinases, and those participating in transcription, inclusive of transctription factors, are believed to be candidates of such drug target proteins. A variety of disciplines of biological research, such as physiology, biochemistry, molecular biology and pharmacology, have contributed to identifying such likely or validated drug targets.

If we are allowed to call the latter as traditional approach, then the genomic/proteomic approach may be called a new one. Perhaps the most efficient is the combination of new and traditional approaches.

Identification of likely or validated target proteins is not the end of the story of drug discovery research. The next step is to select a specific target protein and screen a group of a number of chemical compounds, called chemical compound library, to see if certain compounds modify the function of target protein in a desirable manner. The recently employed process to perform this speedily is called high throughput screening (HTS). The idea is that, by increasing both the number and the degree of diversity of chemical compounds, we would be able to find a good hit that may lead to generation of a new drug that might even be a blockbuster. Here, chemical compounds are compared to arrows. Thus it is the current belief that, if we can increase the kinds and the number of arrows to infinity, at least some arrows will hit the target. Frequently, though, we find ourselves in a position to have discovered no good hit at the completion of such screening, particularly with the chemical compound library available to a pharmaceutical company. It is commonly reasoned that such failure has been due to the limit in number and diversity of available chemical compounds. Combining chemical compound libraries from different sources, including those from the nature, have therefore been tried to enlarge such library in plurality and diversity. It is this inventor's observation, however, that efforts of this kind have not always attained a higher success rate. Recent trend then appears to be such that a pharmaceutical company is trying to bring as many targets as possible into its laboratory and screen their compounds for target after target. So-called biased or focused libraries have been devised to make this kind of efforts hopefully efficient. The questions to be answered are whether this approach will promise a success and, if it does so, how much of success is promised.

### Disclosure of the Invention

Descriptions in this Disclosure of Invention in any combination are drawn to claim construction in this application.

The present invention is based on the recognition that available chemical compounds are limited both in number and diversity to begin with, and that they can never be present in infinity. This recognition may be clear if we consider how many chemical compounds are available to a single pharmaceutical company for drug screening, even after addition of commercially available chemical compound libraries. In a similar vein the presence of a limit in terms of diversity of chemical compound libraries available to a pharmaceutical company is also obvious. We should also note that there is a different sort of restriction in chemical compound libraries. This restriction stems from the concept of drug-likeness that incorporates the idea of drug's toxicity and its availability to the site of action (for review see Clark, D.E. and Picket, S.D. Drug Discovery Today (2000), 5: 49-58). In defining one aspect of drug-likeness, the rule of five, as proposed by Lipinsky, C.A. et al. (Advanced Drug Delivery Reviews (1997) 23: 3-25), is well known. For example, a little overstated here though, one of the rules says that a compound should not exceed 500 in molecular weight to be a drug-like molecule. There are more of demanding restrictions for a molecule to be drug-like. If we think of drug-like molecules only, it may be obvious that, even if pharmaceutical companies altogether worldwide are considered, chemical compounds to be used for screening are limited in number and diversity. An important fact to be recognized in this context is that known drugs approved by health authorities for therapeutic use have historically met the requirements for drug-likeness (with a few exceptions found notably in antibiotics).

The gist of this invention lies in the reversal in the role of arrows and targets. Here, arrows, i.e., chemical compounds, assume the role of targets and, conversely, targets, i.e., proteins, assume the role of arrows. Chemical compounds are regarded as more valued, because of their known structures and of their limited availability, than proteins of unknown function with seemingly limitless future availability in view of the present knowledge of genomics and proteomics. More specifically, drug-like chemical compounds are more valued than proteins. Most valued as target compounds are then those drugs approved for therapeutic use because, as mentioned earlier, a great majority of them satisfy the requirements for drug-likeness. In this scheme a variety of proteins, to be collectively called a protein library, are simultaneously tested for their affinity for each of a selection of target chemical compounds, frequently referred in this specification to as target compounds. Such a protein library can be biased or focused with respect to class, activity, or localization of constituent proteins. With respect to localization, as distinction between such cellular loci as cell surface, cytoplasm and nucleus, may be important, only a cell surface protein library, for example, is constructed. If methods are available, it is possible to construct a more focused protein library such as consisting of all GPCR (G protein-coupled receptor) proteins of a specific cell. A highly focused library can thus be constructed by combination of certain class or activity (such as GPCR) and localization (such as specific cell) of proteins. While the molecular weight of chemical compounds to be studied can be less than 500, according to the rule of five of Lipinsky as described previously, this is extended to less than 600, 1,000, or 1,600 because the restriction in terms of molecular weight is not absolute. Also only a certain portion in structure of a chemical compound that is larger than a fixed value (such as 600) in molecular weight can be responsible for interaction with proteins and therefore we want to identify the partial structure of that portion of the chemical structure as well. This is another reason for extending the restriction in molecular weight. The upper limit in molecular weight of 1,600 is introduced because most of drugs approved for medical use fall within the range of 50 - 1,600 (Hirayama, N, personal communication).

Next, those proteins of desired affinity and specificity toward the target compound are selected and characterized first with respect to their structure (for example, amino acid sequence) and second with their function, most conveniently through survey of appropriate databases such as of NCBI and EMBL. Given certain prior knowledge, experimental characterization of the function of such proteins is also feasible. In this manner we can identify anew one of those proteins to be an interesting therapeutic target to pursue. Because we already know that the particular compound Xₒ, standing for the originator, has a certain degree of affinity and specificity with respect to that protein, the structure of Xₒ is examined and, based upon such examination, attempts can ensue at optimizing affinity, activity and specificity of Xₒ through chemical modification to discover a drug with an entirely new mechanism of action. It is quite likely that a well known drug with known target protein is found to have certain degree of affinity toward other proteins and that one of such other proteins is a distinctly different therapeutic target that may be unthinkable from prior information. Although observed affinity and specificity are elements of importance, consideration must also be given as to if a room is left for optimization by chemical modification of X₀.

As the knowledge from the genomic/proteomic research illustrated above is accumulated, the opportunities for identifying more and more of proteins that are attractive as therapeutic targets are expected to increase. A fact of particular note is that full-length cDNA molecules that encode fully functional proteins will become known or available increasingly in number and diversity in the near future. Also, if an individual or a company has in hand a proprietary database that covers a variety of interactions between chemical compounds and proteins, even if the function of the latter is unknown at the time data are collected, that individual or company may be promised to have a competitive edge over others. This is because, once the function of a certain protein included in the proprietary database becomes characterized and turns out to be very attractive, the individual or company can be ready to start a process of optimizing the originator Xₒ to obtain a new drug of value or can already have a real or virtual pool of compounds, each having or being expected to have desired levels of affinity and specificity for the protein, from which to select a suitable compound as a drug.

Further, if we select a small molecule compound and if we envisage a situation where we can have access to the majority of proteins existing in this world, the above-mentioned approach would yield a catalog or database of almost all proteins that bind this compound. If such selected compound (X₀) is a known drug that has been approved for therapeutic (i.e., medical) use and if those proteins are of human or mammalian origin, such a catalog or database would list almost all of candidate drug target proteins toward which X₀ can be optimized for affinity and specificity by chemical modification. The increasing availability of full-length cDNA molecules that encode human or mammalian proteins make this approach realistic. In addition, it is possible to use cell lysate, whether fractionated or unfractionated, with which to expand and perfect accessible protein source.

It is also possible that one of those proteins turns out to be a protein responsible for certain toxicity or adverse reaction of X₀. Here, X₀ is not necessarily a known drug but can be a compound obtained during drug discovery research. If this is observed, X₀ is minimized with respect to affinity for that protein by chemical modification to yield a better drug compound with desired specificity and affinity for therapeutic target protein but with reduced toxicity or adverse reaction. This approach can be extended to toxic industrial or environmental chemical compounds. The affinity-based survey of almost all proteins existing in this world would help identify those proteins responsible for the toxicity of these substances. When such proteins are identified, measures can be taken to reduce industrial or environmental hazard, for example, by finding an appropriate substitute that has reduced affinity for these proteins, for example, by chemical modification of the toxic substance.

Still further, in addition to access to almost all human or mammalian proteins existing in this world, if we select all of known drugs that are approved for therapeutic use as X₀s, we obtain a good opportunity for securing an unimaginably large pool of drug target proteins toward each of which corresponding X₀ can be optimized for affinity and specificity by chemical modification to obtain quite a large number of better new drugs or previously unthinkable new drugs. Note that these approved drugs are those compounds that have satisfied the requirements for drug-likeness. It may be that this approach ends up with identification of almost all of potential drug target proteins that are of human or mammalian origin since a long history of drug discovery might already have been able to identify almost all of essential chemical structures that satisfy the requirements for a compound to be qualified as a drug. Such identification produces a catalog or database of almost all potential drug target proteins.

The advance in computational chemical synthesis technology would further enable listing of almost all of virtually synthesized drug-like compounds that are derivable from X₀s. This would then mean that the approach described above could in the end identify almost all of chemical compounds, regardless of whether presently known or unknown, that are potentially useful as drugs. Again, a catalog or database can be formed. With the increasing number of approved drugs, by adding them to the list of X₀s to be evaluated from time to time, this approach is expected to further aid the discovery of new valuable drugs.

The whole of the above and subsequent description of interactions between proteins and chemical compounds equally applies to interactions of portions, regardless of whether those portions are isolated as peptides or not, of proteins characterized by such expressions as domains, motifs, ligands, ligand portions, fragments, peptides and polypeptides. Here a portion in singular form means a domain, motif, ligand, ligand portion, fragment, peptide, or polypeptide, all in corresponding singular form. While full-length cDNA molecules are potentially capable of yielding corresponding functional proteins, cDNA molecules that are not of full-length are also important as source for such portions of proteins. In addition, the whole of the above and subsequent description of interactions between proteins and chemical compounds equally applies to interactions of proteins modified post-translationally, or as a result of protein-protein interaction(s), or otherwise.

Instead of selecting all of approved drugs, we can also select representative drugs. This approach is expected to reduce redundancy in the work to secure a good quality pool of drug target proteins by the processes of affinity evaluation outlined thus far. Representative drugs can be selected on the basis of chemical structure, mechanism of action, pharmacological effect, or disease or symptom for which a drug is indicated. For example, the term minor tranquilizers denote compounds with anti-anxiety activity. These drugs consist of groups of compounds with different chemical structures. A group of them are classified into benzodiazepines. A representative drug here may be diazepam. Therefore, instead of testing all of approved benzodiazepine drugs, we may want to select diazepam as representing minor tranquilizers of benzodiazepine class for use in affinity evaluation. H₂ blockers present a difficult case in selecting a representative compound because, while chemical modification originally started from histamine, continuous efforts to improve the pharmacological profile resulted in compounds of a variety of structures that were no more akin obviously to histamine in the end. In such a case, we may want to test a majority of approved drugs in that class.

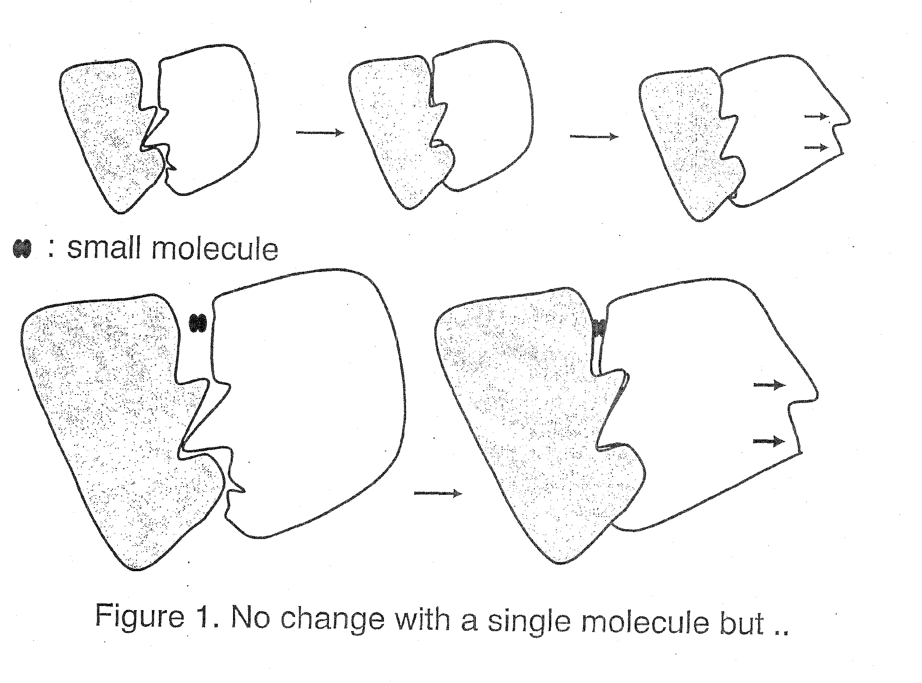

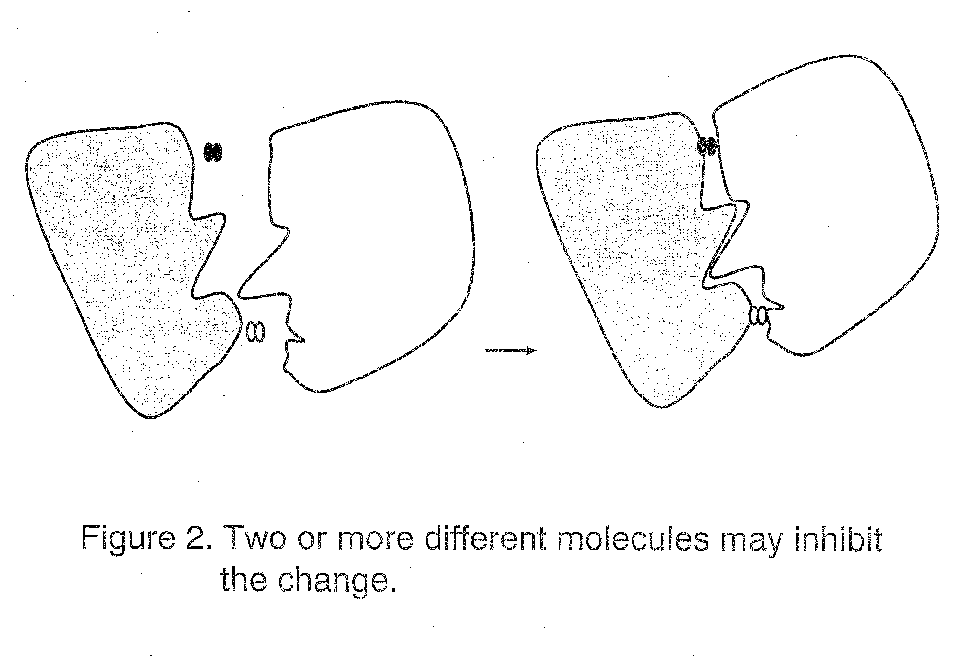

Usually, it is difficult to intervene or modify a protein-protein interaction with a single small molecule compound because such interaction is the result of the contact of the pair of proteins over too large a surface area on both sides of proteins for the compound to cover. If, however, a group of two or more different compounds are found to bind to different sites on the contact surface of at least one of the pair of partner proteins, where each compound binds to the same or a different partner protein, it may be possible to effectively intervene or modify the protein-protein interaction by therapeutically using a combination of such compounds. Figures 1 and 2 illustrate this principle. The upper part of Figure 1 shows a protein-protein interaction that results, for example, in morphological change of the protein on the right hand side (see nose and jaw-like protrusions on the back of the head-like structure) that may cause an effect or lead to another set of protein-protein interaction. The lower part of Figure 1 then illustrates that a single small molecule compound is unable to affect the interaction. As shown in Figure 2, however, with the use of two different compounds having different sites of attachment, the interaction is inhibited from occurrence. It is possible to intervene or modify protein-protein interaction without attachment of a compound to a site on the interacting surface but by modification of configuration of one of the proteins in an allosteric manner through attachment to a site not situated on the interacting surface. A combinatorial therapeutic use of different compounds with different sites of attachment, whether on the interacting surface or elsewhere, can in principle induce intervention or modification of protein-protein interaction more effectively.

The approach described in this invention enables identification of what combination of compounds is to be evaluated for its ability to intervene or modify a set of protein-protein interaction since the approach gives information on what compound attaches to each of the partner proteins involved in the interaction. Again, such identification enables formulation of a catalog or database. To be cautioned in this type of evaluation, however, is the phenomenon of competition for attachment to the same or similar site, such competition potentially resulting in reduction in the interventional or modifying effect of one or more of evaluated compounds.

In a preceding paragraph of this specification, it is described that there is a possibility of modifying protein-protein interaction without attachment of a compound to a site located on the interacting surface but by modification of configuration of one of the proteins in an allosteric manner through attachment to a site not located on the interacting surface. This aspect is further pursued in the subsequent paragraphs without limiting ourselves to protein-protein interactions.

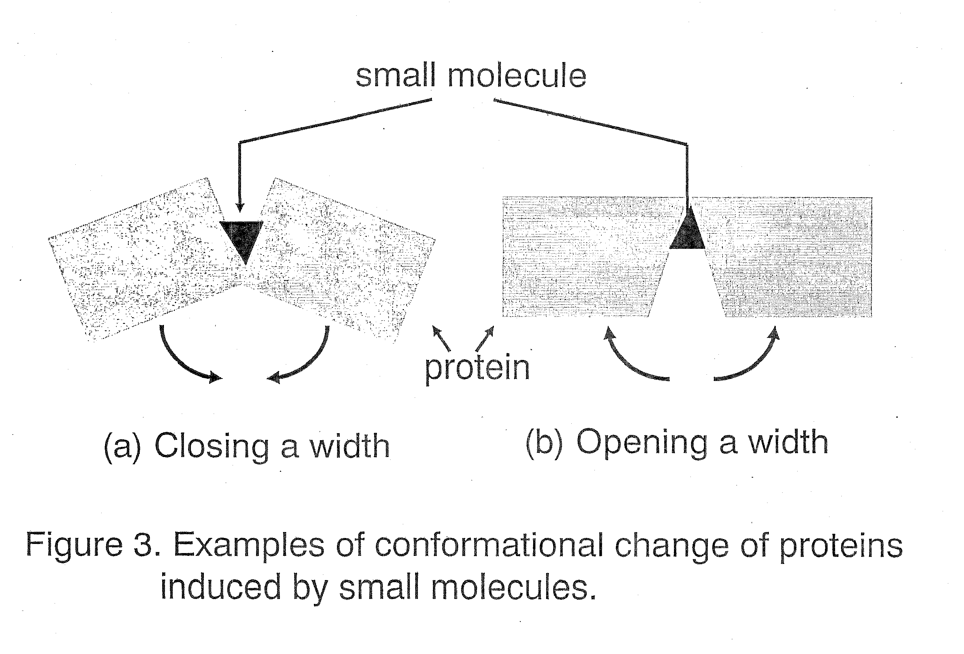

The conformation of a protein molecule can be modified by interaction with small molecules in a variety of manners. For example, a chemical compound can act as an obstacle to the movement of a movable structure of a protein or a portion of a protein. Such a movable structure is not necessarily in direct association with so-called active site. Figure 3 illustrates examples of such modification by a small molecule that acts as a wedge inserted into a hinge-like or joint-like structure of the protein molecule. Thus, a small molecule can close (i.e., narrow) a width (gap) (Figure 3. [a]). A small molecule can open (broaden) a width (gap)(Figure 3. [b]). Modification of this type can induce enhancement or inhibition of the function of a target protein. If a protein is functionally damaged, for example, by mutation in a certain part of amino acid sequence and further if this damage is a result of narrowing of a gap that is necessary for protein's normal function, a small molecule acting in mode [b] would be effective in restoring its normal function by broadening the gap. This and other types of conformational modification by small molecules are in turn expected to produce enhancement, restoration, and inhibition of a chain of protein-protein interactions.

The types of conformational modification described in the preceding paragraphs are not limited to those produced by a single molecule. A combination of several different molecules can in concert produce a desired conformational change by attaching to different sites of a protein within or near the hinge-like or joint-like structure that normally allows the movement of the protein.

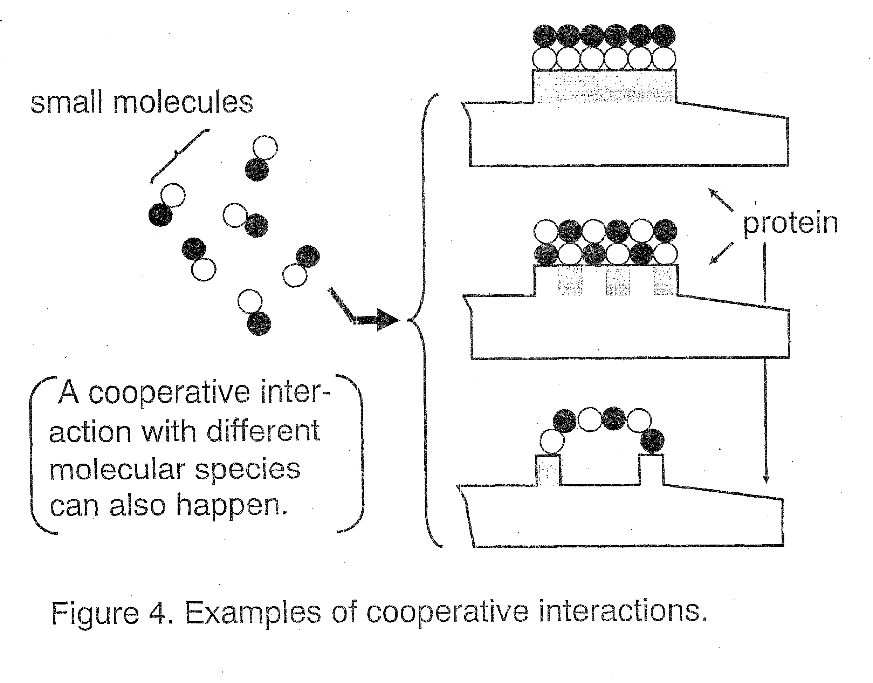

In terms of a combination of multiple, as opposed to single, small molecules, so-called "cooperative interaction" should also be considered. Figure 4 illustrates examples of cooperative interactions where the same small molecular species are shown. As parenthesized, a cooperative interaction can also occur with a mixture of different species of small molecules. Here we call the interaction of a constituent single molecule with a site on a protein molecule as unit interaction. Thus, even if such unit interaction is weak, such a mixture of same or different small molecular species can have a strong interaction (binding) with a protein molecule as a whole due to cooperative interaction. The exploration of small molecule-protein interaction described in this specification can discover a variety of unit interactions. The exploration of small molecule-protein interaction described in this specification can also discover a variety of cooperative interactions brought by a number of molecules of a single, as opposed to different, molecular species. The latter becomes obvious by finding a sharp rise in binding in an affinity parameter-versus-concentration curve where the concentration of the protein is kept constant but that of the small molecule (or those concentrations of different molecules) being studied is varied. Furthermore, by combining weak unit interactions due to different small molecular species as discovered in an initial study, it is possible to obtain a stronger cooperative interaction with a particular protein.

An example of the inhibition of the function of a protein by a compound through inhibition of its movement is the interaction of polyoxometalates with the hinge-like structure of HIV-1 protease (Judd, D.A., et al. J. Am. Chem. Soc. (2001) 123: 886-897). Although the compounds studied, polyoxometalates, are large in molecular weight, i.e., about 4,500, the principle of inhibition of hinge motion by a much smaller molecule is considered to still apply. Another example of induction of conformational change is a molecular brace that reportedly restored the function of mutant p53 by enabling it to bind DNA (Foster, B.A., et al. Science (1999): 286, 2507-2510). In this study greater than 100,000 synthetic compounds were screened and multiple classes of small molecules (300 to 500 daltons) were found effective in the screening. While one of these compounds, CP-31398, was found to effectively inhibit the growth of small human tumor xenografts with naturally mutated p53 at daily doses of 100 mg kg⁻¹, it is unclear from the concentration-response data of a reporter gene cellular assay if such inhibition involved a type of cooperative interaction.

This invention includes the method of exploring cell surface proteins. These proteins are frequently sensitive in their function to conformational change and, for this reason, it is desired to obtain an interaction between a chemical compound and a cell surface protein in such an intact state as it is present on the cell surface. Therefore, included in this invention are cases where cells as such are used as the carrier of a particular cell surface protein.

This invention also includes the method of exploring proteins associated with intracellular as well as cell surface membranous structures. A protein associated with membrane is sensitive in their function to conformational change and therefore it is desired again to observe an interaction of a chemical compound with such an intact protein as it is associated with cellular membrane. Therefore, included in this invention are cases where extracellular virions are used as the carrier of a particular membrane-associated protein.

A membrane-associated protein can also be obtained physico-chemically by treatment of cells with a solution containing a mild detergent or a mixture of mild detergents.

A note of caution is warranted here. Recognizably the approach taken in this invention is primarily affinity-based. It should be understood that a high degree of affinity of a compound for target protein does not necessarily assure the presence of an effect in modifying the function of the latter. For instance, if it is desired to find an inhibitor of certain function of target protein, it will be necessary to further construct a biological assay system where its inhibitory action can be ascertained. Such an assay system may be cell-based, tissue-based, organ-based or whole animal-based. It is recommended to additionally use an appropriate set of such assay systems.

When a compound is found to bind to a limited number of specific proteins with relatively high association constants (i.e., with certain degrees of specificity and affinity), we want to know if such binding is biologically significant. The same applies when a group of compounds sharing affinities for certain proteins are combined and used to modulate the function of each of the proteins. Particularly, we may want to know if a combination of compounds that share affinities for one or both of partner proteins of a protein-protein interaction produces a meaningful outcome in modulating the function of the biological system. One way of knowing if such chemical compound-protein interaction is biologically significant is illustrated in the example below.

Once a chemical compound-protein interaction is found to be biologically significant, it is concluded that the chemical compound involved in the interaction is either stimulatory acting as agonist, or inhibitory acting as antagonist, depending on the function of the protein involved in the interaction. It is then possible to construct a number of screening methods, regardless of whether high-throughput or otherwise, where the protein involved in the interaction assumes the role of a new drug target. These screening methods include affinity assay such as disclosed in this invention and those utilizing cell-based, tissue-based, organ-based, and whole animal-based systems, separately or in a combined manner. When the function of the protein is known or becomes known, appropriate assay methods are devised using a functional indicator such as extracellular, as well as intracellular, pH, extracellular, as well as intracellular, concentrations of calcium, cyclic AMP and other biologically relevant substances, optical change, morphological change and electrophysiological change to ascertain if each of those compounds that interact with the protein in question acts as agonist or as antagonist. A functional indicator is defined by any indicator of the activity of the protein in question regardless of whether it is indicated in cell-free or cellular system. Several examples of ways to learn if a chemical compound acts as agonist or antagonist are presented in Example 10 below, including the use of an antisense molecule (AS) in expression profiling at mRNA level. If an expression profile demonstrated by the chemical compound is found to be similar to that demonstrated by the AS corresponding to the protein, it is presumed that the chemical compound acts as an antagonist to the protein. If the profile is found to be reverse in direction, i.e., for example, up-regulation instead of down-regulation of certain genes, it is presumed that the chemical compound acts as an agonist. This and other processes then result in means to classify compounds into either agonist or antagonist.

The following reviews the meanings of affinity data.

First, let us think about what will be inferred from a set of affinity data. Suppose a set of affinity data particularly with respect to a compound denoted C. Also assume that we have a means to prove whether or not a particular pair of protein-small molecule interaction has a biological significance. Some of such means are described under Example. We divide such interactions into two classes, B (broad) and L (limited). In Class B interactions, the compound C has affinities for a large number of various proteins. In Class L interactions, C has affinities for only a limited number or classes of proteins. Now we form a 2x2 matrix based on the affinity as defined by association constant(s) and on the presence or absence of biological significance in each of the interactions (Table 1).

Let us consider Class B interactions. If C binds to a large number of proteins irrespective of their classes and if association constants observed are large, and further if the majority of such interactions bear biological significance without specificity, we infer that C would be highly toxic. If, however, none of such bindings bear biological significance, then, C would not be effective as a drug when given to humans and simply would distribute itself in the body rather ubiquitously. When association constants are small but such associations have certain biological significance, we would infer that the chances for C to become a drug are negligible. When association constants are small and such associations bear no biological significance, we would conclude that the chances for C to become a drug are also negligible.

Next, we consider Class L interactions. If C binds only to a limited number or classes of proteins and if association constants are large, and further if such interactions bear biological significance, we infer that there would be much chances for C to be either an efficacious drug or a toxic substance. If, however, none of such interactions bear biological significance, then, C would neither be effective as a drug nor would be hazardous as a toxic substance when taken by humans. A particular caution is necessary when C binds only to a limited number or classes of proteins but when association constants are small, and yet when such associations have biological significance. In this case we infer that there would be a chance for us to be able to obtain a good drug by an attempt through chemical modification of C to increase the association constant(s) for a particular protein or a desired class of proteins (refinement with respect to both specificity and affinity). When C is environmentally hazardous, in order to reduce its toxicity, chemical modifications opposite in direction would be appropriate. Finally when association constants are small and when none of the interactions bear biological significance, C would neither be a drug nor a toxic substance.

Further, if an interaction (i.e., binding) of a chemical compound with a protein is found biologically significant and if the function of the protein involved in the interaction is or becomes known, the following is enabled:
(1) Defining the pharmacological activity or toxicity of the chemical compound.
(2) Refining the compound by chemical modification so that specificity and affinity are optimized. Note that this does not necessarily require knowledge on the function of proteins.
(3) Predicting the pharmacological activity and toxicity of a test substance based on a model matrix that is formulated with the use of data on the interactions between known compounds and known proteins as illustrated in Table 2. Thus, there is a method of predicting the pharmacological activity and toxicity of a test chemical compound where the affinity profile of the test chemical compound is compared with a model matrix of affinity profiles that is formulated with the use of data on the interactions between known compounds and known proteins. Similarly note that this does not necessarily require knowledge on the function of proteins.

Additional aspects of interactions between chemical compounds and proteins are described subsequently. New methods devised for evaluating such interactions are also described.

Recent studies have revealed a striking feature of biochemistry that is occurring in the cell. A typical example is the apparatus for transcription where there is formation of a very large complex of proteins. In a eukaryotic cell, for RNA polymerase II to initiate its work of transcription to form primary RNA transcript from genomic DNA, a variety of regulatory proteins collectively called transcription factors need to cooperate and form quite a large complex. One type of such complex involving enhancer is called "enhanceosome" (Lewin, B., Genes VII, p 639, Oxford University Press, 2,000). Chromatin remodeling is also known to require the formation of a large protein complex. There is evidence that signal transduction pathway is not actually a pathway but rather formation of a large complex constructed by (probably sequential) binding of different proteins and/or of different pre-formed protein complexes. (In this context, for example, even each monomer forming a homodimer is called "different" from each other.) For example, it has been found that TAK 1, acting as bait, pulls down a complex consisting of more than 20 different proteins including TAK 1, the bait, under stimulation of a cell with TGF β (Natsume, T., personal communication). The significance of a protein-small molecule interaction as disclosed in this invention should then be considered in this perspective. Binding of a small molecule to a protein may inhibit or strengthen binding of that protein to another protein, which in turn may affect the formation of a larger complex that occurs in natural state. Also, each of different small molecules may bind to different proteins that are constituents of a complex, resulting in inhibition or enhancement of the function of this protein complex. Perhaps a combinatorial use of different small molecules, each molecular species binding to each of different proteins, is more effective in altering the function of the protein complex than use of a single molecule that affects only the interaction of a protein with another protein. Such a combinatorial use of different small molecules, each molecular species binding to each of different proteins of the complex in a biologically significant manner, can be extended to therapy of certain diseases.

This kind of consideration brings two effects to this invention; one is on the method to evaluate protein-small molecule interaction and the other is on the method to evaluate biological significance of a particular protein-small molecule interaction.

With respect to the method to evaluate protein-small molecule interaction, when a chemical compound is selected fore valuation, it is allowed to interact with a pre-formed complex or with a mixture of proteins that are to form a complex. In the latter case, it is possible to initiate the formation of the complex either by adding a component protein needed for complex formation to the assay system or by adding a reagent needed for complex formation. An example of the latter is exogenous addition of ATP when a kinase is involved in the complex formation. This mode of evaluation can be carried out with an in vitro system where each of proteins participating in complex formation has been completely or partially purified. This mode may be termed a reconstructive experiment. The use of a cell lysate still is a reconstructive experiment. The presence or absence of interaction and its quantitative aspect, if interaction is present, is monitored by a variety of means as described under Examples, including the use of surface plasmon resonance technology.

Another mode of evaluation is to utilize a cell as such, i.e., an in vivo mode. In the previously cited study of Natsume, TAK 1 gene was fused first with calmodulin gene and then further with Protein A gene through a linker sequence coding for a peptide which can be cleaved by a peptidase specific for the peptide. This fused gene was connected with an appropriate vector sequence and was used to transfect a cell. A fused protein corresponding to the fused gene was expressed in the cell. The cell was then stimulated by TGF β. It was expected that a protein complex formed with the fused protein that contained TAK 1 as a "domain." The cell was lysed. The assumed complex was pulled down by the use of an appropriate affinity chromatography first for Protein A, and, after the linker peptide being cleaved, a second affinity chromatography for calmodulin. Such proteins or polypeptides as Protein A and calmodulin are called "affinity hooks" in this invention because they serve as specific hooks for affinity chromatography. Some call this mode of purification "tandem affinity purification." The purified assumed complex was subjected to nano-scale liquid chromatography-electrospray ionization-tandem mass analysis (nanoLC-ESI-MS/MS). This analysis indeed found that a complex consisting of more than 20 proteins was formed. This experiment illustrates an example of how to use a cell in evaluating protein-small molecule interaction. Thus such a cell is first treated with a selected chemical compound and then a protocol similar to the one used by Natsume is followed. If there is a difference in the protein composition of the pulled down complex (that could even be a single molecule but not a complex) from that obtained in the absence of the chemical compound, we conclude that there is a direct interaction between the small molecule and at least one of the proteins or a pair of proteins participating in the formation of the complex, or an indirect effect of the small molecule on the formation of the complex. A single or multiple series of reconstructive experiments are then performed to distinguish between the direct and indirect cases and to identify the protein(s) involved in the interaction with the small molecule. There may in addition be a mixed mode that is in part reconstructive, in part in vivo.

With respect to the method to determine the presence or absence of biological significance of a particular protein-small molecule interaction, the finding in the evaluation using a cell outlined above (in vivo) of a difference in the protein composition of the pulled down complex in the presence and absence of the selected chemical compound, if at least one of participating proteins is known to interact with it, directly serves as positive indication for the presence of biological significance. To learn how and in what respect it is biologically significant may require an additional knowledge or information.

The use of a cell as such can be extended to evaluation of protein-small molecule interactions under a different context. A cell is first transfected with an appropriate vector carrying a gene with a tag (termed tagged gene). A histidine tag is one example. The resulting cell is expected to have expressed the protein with that tag and is treated with a selected chemical compound. The cell is lysed after the treatment. Cell lysate, directly or after appropriate step(s) of purification, is subjected to affinity separation, batch-wise or by chromatography, for the tag under the condition where dissociation of the chemical compound from protein is avoided. To avoid dissociation of the chemical compound a physiological condition or a condition close to it is preferred. The eluate, in which the chemical compound-protein association is no more necessary, is then subjected to mass analysis. The resulting mass spectrum is compared with that obtained in the absence of the treatment. As this procedure produces mass spectra of both protein and chemical compound and because they demonstrate the quantities of the two components, quantitative nature, as well as qualitative aspect, of interaction can be studied. Also, the cell that has expressed the tagged protein can be treated with a mixture of chemical compounds. Comparison of mass spectra again yields information as to what chemical compound interacts with the tagged protein and to what extent it interact with the latter. The advantage of this method lies in its ability of identifying an interaction under a condition that closely mimics the natural environment. Natural protein folding is expected in the majority of cases, despite tagging. It is possible under this scheme to identify an interaction of a chemical compound with an intracellularly modified protein, including one that is post-translationally modified. It is further possible to identify an interaction of a chemical compound with a protein complex containing the tagged protein as participant.

The kinds of data to be collected for formulating databases or catalogues are summarized as follows:
(1) Basic data
   - Cᵢ:: Compound i (a modified compound is counted as different)
   - Pⱼ:: Protein j (a post-translationally or otherwise modified protein is counted as different and the same protein prepared differently is counted also as different; portion of a protein also is counted as different)
   - Eₖ:: Environment k of affinity determination (method of affinity determination, solvents, pH, ionic strength, intracellular, cell membrane-associated, etc.)
   - Aᵢⱼₖ:: Affinity determined (any of kinetic, equilibrium, quantitative, semi-quantitative, qualitative, etc.)
(2) Structural data
   - SC_{¡}:: Chemical structure of Cᵢ (1D-, 2D- or 3D-; D stands for dimensional.)
   - SPⱼ:: Structure of Pⱼ (1D-, 2D- or 3D-)
   - SCᵢₖ:: Structure of Cᵢ under environment k
   - SPⱼₖ:: Structure of Pⱼ under environment k
(3) Other attributes (subscripts omitted)
   - FC, FP:: Function (FC could be pharmacological activity, toxicity and side effects of a chemical compound, and the disease or condition a chemical compound is indicated for)
   - GC, GP:: How C or P was *gained* (i.e., method of preparation, etc.)
   - TC, TP:: Target protein for C or P when known (target protein for C or P means a protein that C or P directly interact with, respectively)
   - MC, MP:: Miscellaneous attributes other than above (these can be further sub-categorized and denoted separately)

The following are steps for formulating databases and predictions:

### First Step: Alignment of Aᵢⱼₖ Data and Comparison

1. Alignment of Aᵢⱼₖ data of proteins with affinity values higher than a predetermined level for a compound Cᵢ and comparison of structures of those proteins.
2. Alignment of Aᵢⱼₖ data of compounds with affinity values higher than a predetermined level for a protein Pⱼ and comparison of structures of those compounds.
3. Clustering and alignment of Aᵢⱼₖ data with respect to compounds and proteins:
   1) by ignoring whether or not each of the compounds has been chemically modified for purpose of affinity determination.
   2) by ignoring the difference in the method of preparation (including synthesis and extraction) of the compounds.
   3) by ignoring whether or not each of the proteins has been modified post-translationally, or through protein-protein interactions, or otherwise.
   4) by ignoring the difference in the method of preparation of the proteins.
   5) by ignoring the difference in the environment (condition) in affinity determination.
   6) according to common structures and biological functions with respect to the compounds.
   7) according to common structures and biological functions with respect to the proteins.
   8) by combining any of the above.

### Second Step: Discovery of consensus partial sequence and consensus partial structure with respect to proteins and compounds, including discovery of consensus-equivalent partial sequence and consehsus-equivaleht partial structure

The aligned data obtained in the first step is surveyed visually and/or by use of an appropriate computational program for consensus partial sequence and consensus partial structure with respect to proteins and compounds. This process includes survey for consensus-equivalent partial sequence and consensus-equivalent partial structure. By consensus-equivalent it is meant that a portion of, for instance, amino acid residues of proteins being compared can be exchanged to a different stretch of amino acid residue(s) without significant loss of anticipated functionality and that such stretches are deemed equivalent to each other. The change of leucine to isoleucine is one example. To carry out this type of amino acid substitution, Dayhoff percent accepted mutation matrix 250 (PAM250), blosum substitution matrix 62 (BLOSUM62), or the like can be utilized. As equivalence is not an absolute term, it is possible to define the degree of equivalence by a fixed score value as provided by these matrices. The consideration of equivalence is not limited to comparison of local sequences but is extended to comparison of 3D structures, i.e., positioning of structural elements in space. Therefore, when an amino acid sequence takes an identical or similar 3D structure to that is taken by the other amino acid sequence with identical or similar effects in terms, for example, of mass of occupation, van der Waals force, hydrogen bonding, and electrostatic force, these two sequences are termed consensus-equivalent. The concept of equivalence is also applied to comparison of different chemical compounds. This comparison of chemical compounds includes that of not only 1D or 2D structure but also of 3D structure. In other parts of this specification the terms "common" and "similar" are also used to mean consensus and consensus-equivalent, respectively.

This second step is based on the following assumptions:
1) The sites on proteins, as represented by partial sequences and partial structures of the proteins, responsible for binding to small molecules are limited in number and diversity. These sequences can be identified in amino acid sequence as a single stretch in a location or as multiple isolated stretches in different locations.
2) The sites on compounds, as represented by partial structures, skeletons, and other structural features of the compounds, responsible for binding to proteins are also limited in number and diversity.

In preceding paragraphs, it was described that a single molecule or a combination of multiple same or different molecules can produce a desired conformational change by attaching to a site or sites of a protein within or near the hinge-like or joint-like structure that normally allows the movement of the protein. One may discover consensus partial amino acid sequence(s) located in such site or sites on a protein within or near the hinge-like or joint-like structure. The hinge-like or joint-like structures of certain proteins have been identified, such as in HIV-1 protease (Judd, D.A., et al. J. Am. Chem. Soc. (2001) 123: 886-897). The progress in structural analysis of proteins is expected to enable further elucidation of such movable structures with attendant knowledge of responsible amino acid sequences. Once some of consensus sequences discovered in this Second Step are found to correspond to the amino acid sequences responsible for the movable structures, it is possible to design more desirable compounds, acting through modification of conformational change, for inhibition, restoration or enhancement of the function of the target protein based on previously obtained data of protein-small molecule interactions.

### Third Step: Validating the findings of the second step above and discovering critical partial structures and skeletons with respect to proteins and compounds

This third step is accomplished by the following:
1) Validation - Study changes in Aᵢⱼₖ under gradual chemical modification or the compound in question by reduction in size, substitution, or expansion in size. Also study changes in Aᵢⱼₖ under graded mutation, i.e., substitution of amino acid residue(s) of the protein in question.
2) Discovery of critical partial structures, skeletons and 3D structures - Identify them from the findings of 1) above.

The final goal of these steps is to predict the chemical structure of a compound that would maximize affinity and specificity for a selected target protein when we consider the efficacy of a drug. On the other hand, it is to predict the chemical structure of a compound that would minimize affinity and specificity for a selected target protein when we consider toxicity. Such prediction is validated by preparing (e.g., synthesizing) the predicted compound and by experimentally evaluating its affinity for the selected protein and studying biological relevance of such affinity.

Databases, user-interfaces, and methods of utilizing these databases and user-interfaces are described in a more detailed manner in the subsequent paragraphs.

A database is formulated by tabulating description of interaction between a protein or a portion of a protein and a chemical compound, the latter being selected from a population consisting of chemical compounds of less than 1,600, 1,000, 600, or 500 in molecular weight. These chemical compounds may or may not be approved for medical use. Proteins and portions of proteins in such a database may include those derived from cell lysate, prepared artificially by genetic engineering, expressed from full-length cDNA, focused with respect to class, activity such as enzymatic activity and localization such as cell surface, cytoplasm, nucleus, cell type, tissue origin, and organ origin, and association with a membranous structure of a cell, notable examples being GPCRs, those expressed in extracellular virions and those obtained physico-chemically by treatment of cells with a solution containing a mild detergent or a mixture of mild detergents.

In such a database an interaction is defined by presence or absence of such interaction and by a parameter for intensity of affinity (where appropriate, the word affinity is used interchangeably with the word interaction) and/or by mode of interaction and/or by structural element of interaction. The parameter for intensity of affinity includes (a) an association rate constant and/or a dissociation rate constant, and (b) an equilibrium constant of association and/or an equilibrium constant of dissociation. The mode of interaction includes an interaction due to van der Waals force, hydrogen bonding, electrostatic interaction, charge transfer, hydrophobic, hydrophilic and lipophilic interactions, and cooperative binding or cooperative interaction. The structural element of interaction includes site of interaction, structure of site of interaction, interacting group, interacting amino acid residue, interacting atom, interacting surface, and relative position, in 1-, 2-, or 3-dimensional space, of interacting group, interacting amino acid residue, interacting atom and interacting surface.

It is convenient to formulate a database by tabulating description of interaction of each of a multitude of proteins or portions of proteins with a multitude of chemical compounds. Also convenient is to formulate a database by tabulating description of interaction of each of a multitude of chemical compounds with a multitude of proteins or portions of these proteins. Such a collectively formulated database can also include description of a parameter for intensity of affinity and/or mode of interaction and/or structural element of interaction as described previously. Such a database can also include tabulated description of (a) regulatory regions of genomic DNA sequence regulating the expression of the protein participating in the interaction with a chemical compound, and/or (b) binding sites, on genomic DNA sequence, of transcription factors that initiate the transcription of the gene encoding said protein, and/or (c) genes regulated by any of said regulatory regions, and/or (d) proteins encoded by said genes. Regulatory regions of genomic DNA include promoter and enhancer. Such a database can include description of a parameter for intensity of affinity and/or mode of interaction and/or structural element of interaction as described previously. Such a database can also include tabulated description of proteins or portions of proteins the expression of which is affected by administration of any or any combination of chemical compounds in any or any combination of cell-free, cell-based, tissue-based, organ-based, and whole animal-based assay systems.

A database is formulated to additionally describe in tabulated format SNPs (single nucleotide polymorphism markers) located within exons of the gene encoding said protein and/or SNPs located within regulatory regions regulating the gene encoding said protein and/or SNPs located within binding sites, on genomic DNA sequence, of transcription factors that initiate the transcription of the gene encoding said protein. A database is formulated further to describe in tabulated format positions of SNPs located within exons of the gene encoding said protein, and/or types of these SNPs located within exons of the gene encoding said protein, and/or whether or not each of these SNPs causes an alteration of amino acid residue in corresponding protein, and/or the effect of such alteration of amino acid residue on the 3-dimentional structure of the protein and/or on biological function of the protein. Similarly, a databases is formulated to additionally describe in tabulated format positions and/or types of SNPs located within regulatory regions regulating the gene encoding said protein and/or within binding sites, on genomic DNA sequence, of transcription factors that initiate the transcription of the gene encoding said protein.

All of the above-mentioned databases can include tabulated description of splice variant mRNAs transcribed from a gene(s) encoding a protein(s) or portion(s) of such protein(s). These databases can further include tabulated description of RNA sequences of these mRNAs, amino acid sequences translated from these RNA sequences, and/or 3-dimensional structures resulting from folding of the amino acid sequences. The databases of this invention can include attributes of chemical compounds such as their pharmacological activities and clinical indications that are tabulated in the form of a profile. A clinical indication means not only the disease or symptom a chemical compound used for medical purpose is indicated for but also its clinical effect such as acceleration of healing of duodenal ulcer, lowering of plasma cholesterol level, etc. A pharmacological activity can include clinical pharmacological activity that in certain instances may be synonymous to clinical indication. Such a database of pharmacological activity profile, further describing in tabulated format the presence or absence of pharmacological activity and/or the degree of pharmacological activity, can be collectively formulated into another database that accommodates data on a plurality of chemical compounds. Similarly, the databases of this invention can include other attributes of chemical compounds such as their toxicities and adverse side effects that are tabulated in the form of a profile. Toxicity can include clinical toxicity that may be synonymous to an adverse side effect. Such a database of toxicity profile, further describing in tabulated format the presence or absence of toxicity and/or the degree of toxicity, can be collectively formulated into another database that accommodates data on a plurality of chemical compounds. A database is formulated that is characterized by tabulated description of a protein-protein interaction, wherein at least one of proteins participating in the interaction is capable of interacting with a chemical compound of less than 1,600, 1,000, 600, or 500 in molecular weight and/or approved for medical use. A database is formulated that is characterized by tabulated and/or graphical description of networks of interactions among a plurality of proteins or portions of proteins at least one of which is capable of interacting with a chemical compound of less than 1,600, 1,000, 600, or 500 in molecular weight and/or approved for medical use.

A user-interface displaying the output from any or any combination of the above-mentioned databases in tabulated and/or graphical format is constructed.

It is convenient when a method is in hand for searching information on a chemical compound characterized by the use of any or any combination of the above-mentioned databases, concerning proteins or portions of these proteins that interact with the chemical compound, and/or proteins or portions of proteins that are capable of interacting with other proteins or other portions of proteins, and/or proteins or portions of proteins the expression of which is affected by the chemical compound, and/or networks of interactions involving some or all of proteins or portions of proteins and the chemical compound, and/or information pertaining to the chemical compound and to proteins or portions of proteins involved in the networks of interactions.

It is further convenient to construct a user-interface that displays, in tabulated and/or graphical format, the output resulting from the use of the methods described in the preceding paragraphs. Such a user-interface displaying interactions can be made more convenient by expressing as a connecting line a linkage between a chemical compound and a protein or a portion of a protein and as another connecting line a linkage between a protein or a portion of a protein and another protein or another portion of a protein, wherein each of the chemical compounds and proteins or portions of proteins being expressed as a node in networks of interactions. Such a user-interface can be made still more convenient by displaying in the networks of interactions the intensity of interaction, preferably expressed as association and/or dissociation rate constant and/or equilibrium association constant, and the degree of effects of that interaction on the expression of proteins involved in the networks of interactions. These user-interfaces may accommodate information in tabulated and/or graphical format concerning SNPs located within exons of the gene encoding said protein and/or SNPs located within regulatory regions regulating the gene encoding said protein and/or SNPs located within binding sites, on genomic DNA sequence, of transcription factors that initiate the transcription of the gene encoding said protein. These user-interfaces may further accommodate in tabulated and/or graphical format information concerning positions of SNPs located within exons of the gene encoding said protein, and/or types of these SNPs located within exons of the gene encoding said protein, and/or whether or not each of these SNPs causes an alteration of amino acid residue in corresponding protein, aud/or the effect of such alteration of amino acid residue on the 3-dimentional structure of the protein and/or on biological function of the protein. Also, some of these user-interfaces may accommodate in tabulated and/or graphical format information concerning positions and/or types of SNPs located within regulatory regions regulating the gene encoding said protein and/or within binding sites, on genomic DNA sequence, of transcription factors that initiate the transcription of the gene encoding said protein.

It is also convenient when a method is in hand for searching information on a protein or a portion of a protein (collectively denoted "questioned protein") characterized by the use of any or any combination of the above-mentioned databases, concerning chemical compounds that interact with questioned protein, and/or other proteins or other portions of proteins that are capable of interacting with questioned protein, and/or proteins the expression of which is affected by questioned protein, and/or networks of interactions involving part or all of said proteins or said portions of proteins including questioned protein and said chemical compounds, and/or information pertaining to each of chemical compounds involved in the networks and to each of proteins or portions of proteins involved in the networks.

A user-interface is constructed, displaying the output resulting from the use of the method described above in tabulated and/or graphical format.

It is possible to devise a method to search different chemical compounds but with identical or similar profiles in terms of the intensity of interactions, preferably expressed as association and/or dissociation rate constant and/or equilibrium association constant, with proteins or portions of proteins, and/or information pertaining to each of these chemical compounds, when some or some combination of databases and user-interfaces mentioned above are used. Similarly, it is possible to devise a method to search different proteins or different portions of proteins with identical or similar profiles in terms of the intensity of interaction, preferably expressed as association and/or dissociation rate constant and/or equilibrium association constant, with chemical compounds, and/or information pertaining to each of the proteins or portions of proteins, when some or some combination of databases and user-interfaces mentioned above are used.

A user-interface is constructed, displaying the output resulting from the use of the method described above in tabulated and/or graphical format.

It is also possible to devise a method to search different chemical compounds with identical or similar profiles in terms of pharmacological activity and clinical indication, and/or information pertaining to each of such chemical compounds by the use of some or some combination of databases and user-interfaces mentioned above. Similarly, it is possible to devise a method to search different chemical compounds with identical or similar profiles in terms of toxicity and adverse effect and/or information pertaining to each of the chemical compounds by the use of some or some combination of databases and user-interfaces mentioned above.

A user-interface is constructed, displaying the output resulting from the use of the method described above in tabulated and/or graphical format.

It is of course possible to devise a method to search different chemical compounds with identical or similar profiles in terms both of pharmacological activity and toxicity, and/or information pertaining to each of the chemical compounds by the use of some or some combination of databases and user-interfaces mentioned above.

A user-interface is constructed, displaying the output resulting from the use of the method described above in tabulated and/or graphical format.

It is necessary to devise a method of data mining to extract the relationship between (a) the interaction of a chemical compound with proteins or portions of proteins and (b) pharmacological activity, and/or toxicity, of the chemical compound. This is accomplished by comparing profiles, recorded in the previously mentioned databases and user-interfaces, of the chemical compound with respect to interaction with proteins or portions of proteins and to pharmacological activity and/or toxicity, respectively. Such extraction of the relationship can be based on the assumption that those proteins or portions of proteins with high affinities for the chemical compound in question are responsible for its pharmacological activity and/or toxicity. The data on its intensities of affinity for proteins in its profile along with additional information on the function of the protein and on the availability of the protein in particular tissues and cells may be used to identify a protein or proteins responsible for particular pharmacological activity and/or toxicity.

It is also necessary to devise a method of data mining to extract the relationship in structure of (a) chemical compounds and (b) proteins or portions of proteins having affinity for each other. This is accomplished by comparing structural categories (see below for definition) of the chemical compounds and the 1-, 2-, and 3-D structures of the proteins or portions of proteins with profiles of interactions (affinities) that are recorded in databases and user-interfaces mentioned above.

This aspect of data mining is divided into the following three categories and each is described in detail:
(1)A multitude of different chemical compounds having affinity for a single protein (multiple compounds-versus-single protein mode).
(2) A multitude of different proteins having affinity for a single chemical compound (multiple proteins-versus-single compound mode).
(3) A multitude of different chemical compounds each having affinity for each of a multitude of different proteins (multiple-versus-multiple mode).

First is to extract the relationship in structure of (a) a multitude of different chemical compounds, denoted "queried compounds," and (b) a single protein or a single portion of a protein where each of (a) has affinity for (b). This is accomplished by comparing structural categories of the queried compounds and by extracting common or similar structural categories. Databases and user-interfaces mentioned above accommodate some of structural categories as attributes of each chemical compound, but databases and user-interfaces of a different kind may need to be constructed for further convenience. Here, the structural category can mean any category that results from attempts to extract structures or substructures that are common or similar among a group of different chemical compounds. The structural category includes a partial structure or atom such as carboxyl group, amino group and halogen, and a skeleton such as steroid and indol. This may mean inclusion in the structure of a particular homocycle or heterocycle. While the rules of IUPAC and IUPAC-IUB Nomenclature can define such structural categories and are very useful, these rules alone are not sufficient for the purpose of this invention. Thus, a structural category may be defined by localization in space of a particular hydrophobic group of defined size (dimensions) and of shape (sheet, sphere, rod, etc. and their combinations). Relative positions in space of several such hydrophobic groups along with their individual size and shape may be important. The position, relative to that of a hydrophobic group or several hydrophobic groups, of a charged atom or group with defined charge (positive or negative), size and distance that its electrostatic force reaches (electric field) may be important. The length and flexibility of any chain linking different groups are taken into consideration. The rotational freedom is also considered. The presence and relative position of a group(s) capable of hydrogen bonding may be important and this may be extended to the consideration of solvation by water molecule(s). All these and other structural descriptors are combined and may form hierarchy of commonness or similarity shared by different chemical compounds. Such hierarchy may be constructed in several different ways, depending on how one attaches relative order of importance to different structural aspects. It is also possible that combination of structural descriptors results in non-hierarchical structural categories and that these categories are common or similar in different chemical compounds. In other words, commonness or similarity at any level and at any aspect extracted from the structures of a group of different chemical compounds is structural category. Because we want to extract those structural categories that are associated specifically with a group of different chemical compounds having affinity for certain proteins, those that are frequently associated with a random sample of different chemical compounds, termed "nonspecific structural categories," need to be filtered out. This is achieved by extracting common (but not similar) structural categories from a randomly selected sample of compounds. The size of such sample is important. Several samples are used to avoid bias. Collections of nonspecific structural categories are constructed at different levels, depending on sample size, number of random samples used, and characteristics, in terms of diversity of compounds, of each of random samples selected for this purpose. Generally, the larger sample size and larger number of samples result in the fewer extracted nonspecific structural categories. A collection of such fewer extracted categories is termed "collection of low level." The structural category as a term used in this invention excludes nonspecific structural category. Because we do not want to miss structural categories that are associated specifically with selected set of chemical compounds, it is recommended to initially use a collection of low level and increase stepwise the level of collection to filter nonspecific categories out from common or similar structural categories. Clustering is another language meaning the process of dividing a set of entities into subsets in which the members of each subset are common or similar to each other but different from members of other subsets. The Tanimoto's similarity index, the PPh-Triangle method and its variation to a dynamic version, the CoMFA, and other methods have been utilized for this purpose. Aspects of clustering of a number of chemical compounds that uses several structural descriptors have been reviewed (Brown, R. and Martin, Y. C., J. Chem. Inf. Comput. Sci. (1966) 36: 572-584 and ibid., (1997) 37: 1-9). By combining such structural descriptors, there result multidimensional clusters, each cluster sharing a certain structural category. Once such common or similar structural categories are extracted from chemical compounds that share affinity (that is higher than a fixed level) for the protein or portion of protein in question, they become candidates of those structural categories responsible for the interaction of these chemical compounds with that protein or portion of protein. One of the purposes of this kind of data mining is to probe a protein with a variety of structural categories that are presumably responsible for interaction with protein and to characterize it with the use of the queried compounds as "chemical probes." "Chemical probing" of a protein with a multiple of chemical compounds but without relying on *a priori* extraction of common or similar structural categories is described later under (3) through (5) of the story of Cox-1 and Cox-2 substrate and inhibitors. Once strong interactions are found between the protein and each of certain chemical compounds, attempts to extract common or similar structural categories from these compounds can ensue.

Second is the converse of the first and is to extract the relationship in structure of (a) a multitude of different proteins or different portions of proteins, collectively denoted "queried proteins," and (b) a single chemical compound where each of (a) has affinity for (b). This is accomplished first by comparing amino acid sequences of the queried proteins that are recorded in databases and user-interfaces mentioned above. It may be possible to see that some of the queried proteins that share affinity (that is higher than a fixed level) for the compound in question possess a common (consensus) or similar (consensus-equivalent) partial sequence. Such common or similar partial sequences can be found at several locations within the entire length of compared sequences. A chain comprising such common or similar partial sequences and single residues, not necessarily in the same order, may be found in the sequences of different proteins having high affinity for the compound, where the sequence at the linker position is relatively of low importance. It is assumed that such common or similar sequences and residues are, whole or in part, responsible for binding of these proteins or portions of proteins to the compound. It is further assumed that these sequences and residues, whole or in part, form sites in the form of points, ridges and the like (or even a charged cavity to attract or expel part of a small molecule) to suitably lodge the compound on the surface of the proteins or portions of proteins. Depending on the availability of additional structural data on some of the proteins, obtained most reliably by X-ray crystallography analysis of complexes of these proteins with the same or similar chemical compound or least reliably by computational modeling of such complexes, it is also possible to construct a 2- or 3-demensional map of these lodging sites, with identification and characterization of electric fields, sites of hydrogen bonding and van der Waals contacts responsible for molecular association. It is also possible that the structure of the site of binding of small molecule on the proteins is distorted (i.e., strained) to form a pocket and hence thermodynamically unstable but suitable for docking such a small molecule. Examples of binding pockets are those observed in HIV-1 protease (Judd, D.A., et al. J. Am. Chem. Soc. (2001) 123: 886-897) and Cox-2 (Kurumbail, R. G., et al., Nature (1996) 384: 644-648). For certain reason(s) some of these seemingly unstable structures might be actually stable enough and might have been evolutionally conserved to be used by organisms as convenient modules. There may be a certain number of such modules different from each other in structure. These modules must have been limited in number (and therefore in kind) because of the thermodynamic restriction. It is therefore possible that organisms through evolution utilized each of them to construct a number of different proteins. Thus the same module could be found in a number of different proteins of a single species of organism. These proteins having in common the same module may possess similar, related, or different functions. If one places queries for a wide range of proteins having affinity for a small molecule in a single species of organism, these evolutionally conserved modules, each represented by whole or part of the previously described chain comprising common or similar partial sequences and residues, can be identified as commonly participating in the interactions of proteins with that molecule. The chances of such identification will be increased when a similar survey is conducted cross-species, covering a wide range of different species of organisms. Furthermore, it may be possible to construct a 2- or 3-demensional map of the lodging sites for each of the modules with identification and characterization of electric fields, sites of hydrogen bonding and/or van der Waals contacts responsible for the molecular association. "Chemical probing" may enable or help enable all of these.

The last is to extract the relationship in structure of (a) a multitude of different chemical compounds, denoted "queried compounds," and (b) a multitude of different proteins or different portions of proteins, collectively denoted "queried proteins," where each of (a) has affinity for each of (b). This is the data mining of multiple-versus-multiple mode and is the most rewarding application of "chemical probing."

For simplicity, protein means both protein and portion of protein, unless specified otherwise. Part of descriptions on the multiple-versus-multiple data mining here is also relevant to data mining of multiple compounds-versus-single protein mode and that of multiple proteins-versus-single compound mode.

The multiple-versus-multiple data mining starts with extracting common or similar structural categories by comparing structural categories of the queried compounds having affinity, expressed for example by the equilibrium association constant Aᵢⱼ that is greater than a cutoff point A₀, for each of the queried proteins. We then prepare a table listing common or similar structural categories (simply structural categories, hereafter) for each of the queried protein. For example, when protein P₃ is found associated with structural categories H₄, H₇ and H₈ and protein P₅ with H₂, H₇ and H₈, etc., we prepare the following table where the presence of such association is shown by a + sign:

| Str.Ca | H | H | H | H | H | H | H | H | H |
|---|---|---|---|---|---|---|---|---|---|
| t: | 1 | 2 | 3 | 4 | 5 | 6 | 7 | 8 | 9 |
| P₁ | | | | + | | | + | + | |
| P₂ | | | | | | | | | |
| P₃ | | | | + | | | + | + | |
| P₄ | + | | | | | | | | + |
| P₅ | | + | | | | | + | + | |

Notice that P₁ and P₃ show the same profile of association with structural categories H₄, H₇, and H₈, indicating the likelihood of these two proteins having affinity for those compounds represented by the set of structural categories H₄, H₇, and H₈. This is a prediction that can be tested for its validity by studying interactions between each of these proteins and another set of compounds represented by structural categories H₄, H₇, and H₈. Such a prediction is refined for correctness by repeating this procedure. Also important is the prediction that the two proteins have at least one binding site in common for compounds represented by H₄, H₇, and H₈. This prediction is later combined with the findings from the side of protein sequences, yielding a more important and therefore useful prediction. Proteins showing profiles of association similar to each other, such as P₁/P₃ and P₅, may possess binding sites similar to each other and this may serve further analysis to be carried out in conjunction with the findings from the side of protein sequences.

Common (consensus) or similar (consensus-equivalent) partial sequences are extracted in a similar but more complicated manner. We first prepare a table like the one that follows to show the interactions between chemical compounds (Cᵢ) and proteins (Pⱼ), where a + sign indicates the presence of interaction with affinity expressed, for example, by the equilibrium association constant Aᵢⱼ that is greater than a cutoff point A₀:

| | P₁ | P₂ | P₃ | P₄ | P₅ | P₆ | P₇ | P₈ |
|---|---|---|---|---|---|---|---|---|
| C₁ | | + | | | + | | | |
| C₂ | + | | | + | | + | | |
| C₃ | | | + | | | | + | |
| C₄ | | | | | | | | + |
| C₅ | | | + | + | + | + | | |
| C₆ | + | | + | | | | | |

For example, C₂ has affinity for P₁, P₄, and P₆. We compare the amino acid sequences of these proteins to find and extract consensus or consensus-equivalent partial sequences in P₄ and P₆, like [... KISS .. ME .. TENDER] and [... KISS .. ME ... SENDER]. We preliminarily assign these partial sequences to those participating in the interaction of C₂ with P₄ and P₆. (Generally but not absolutely, correctness of assignment would increase with increasing affinity and specificity.) By repeating this with respect to each of other chemical compounds, we find [.. KILL ... HER . TENDER] or an equivalent in the interactions of C₅ with P₃ and P₆, for example. We may find more of such sequences in other sets of interactions. We pick up stretches of continuous amino acid codes (termed "words" and abbreviated to W's) such as KISS (W₁), KILL (W₂), ME (W₃), HER (W₄), and TENDER-SENDER (W₅) found in presumptive interaction-participating sequences and search for these words in all of the sequences of the proteins P₁ through P₈. (Those words resulting from permissible exchange of amino acid residues are counted as the same word.) Retaining the information on the protein origin and the location of each word in the sequence of the protein of origin, we then construct a table such as shown below.

| Word: | W₁ | W₂ | W₃ | W₄ | W₅ | W₆ |
|---|---|---|---|---|---|---|
| C₁ | | | | + | + | + |
| C₂ | + | | + | | + | |
| C₃ | | + | | + | + | |
| C₄ | | | | | | |
| C₅ | | + | | + | + | |

If all members of the word set W₂, W₄, and W₅ coexist in a protein that has affinity for C₃ and C₅ and if the same is true for another protein, and further if the relative locations of these words are similar in these proteins, we preliminarily assign a chain comprising these words as being responsible for interaction of C₃ and C₅ with these proteins and assume that C₃ and C₅ have binding sites that are at least partially identical to each other. This is to assign a chain comprising words coexisting in a protein in similar locations among several proteins as being responsible for a compound-protein interaction. Perhaps a little remote, similar assignment may be made with respect to C₁ and C₃/C₅, if W₄ and W₅ are localized in a protein that has affinity for C₁ as well as for C₃/C₅. This is called "assignment of a chain by incomplete matching of word set." Once such a chain comprising a particular set of words is identified, model proteins bearing similar chains for which crystallographic data are available are searched for. By referring to such data, it is then possible to construct spatial localization of the words, i.e., the 3-dimensional structure of the chain in question.

In picking up common or similar words, we may want to exclude nonspecific words as we previously excluded nonspecific structural categories for chemical compounds. This can be done but should be done with caution. A chain as defined in this invention is like a sentence. It can be understood that frequently appearing words are important in a sentence despite their frequent appearance_{.}

Combining the results of both approaches, one from common or similar structural categories of chemical compounds and the other from common and similar sequences of proteins together with their 3-dimensional structures, is expected to yield the most rewarding inferences. To simplify the discussion, we consider an interaction between a particular pair of chemical compound and protein for which abundant surrounding data have been obtained by evaluation of interactions of other chemical compound-protein pairs to support its structural aspects and modes. Under these circumstances it is highly likely that both identified structural categories in the chemical compound and identified partial sequences of the protein together with their 3-dimensional structures are responsible for this interaction. The high likelihood itself is of value. But more valuable is greater certainty with which one can identify a newly found protein as having affinity for the compound, if it is found to have the same or similar partial sequences as the one already identified. Still more valuable is the ease with which one can design the structure of a chemical compound that has a higher affinity for the specific site of binding, based on the structural categories defined from foregoing analyses and 3-dimensional structures of interaction-participating chains. Characterization, with the help of crystallographic data, of the binding site with respect to electric fields, sites of hydrogen bonding and/or van der Waals contacts would facilitate such designing. This is a great advance from current practice of more or less trial-and-error nature, particularly in the field of drug design.

The story of Cox-1 and Cox-2 substrate and inhibitors gives insights into the analyses described above. Arachidonic acid is the substrate for both Cox-1 and Cox-2. Non-steroidal anti-inflammatory drugs (NSAIDs) act at the cyclooxygenase active site of both Cox-1 and Cox-2 without much specificity, causing gastric side effects. By contrast, several Cox-2-seiective inhibitors have been identified with potent anti-inflammatory activity but with minimal gastric side effects. The two enzymes show a sequence identity of about 60% and the overall 3-dimensional structures are highly conserved. These facts along with the discussion on evolutionally conserved modules described previously show several things. (1) Small molecules of apparently different structures (such as arachidonic acid, NSAIDs such as flubiprofen and indomethacine, and a Cox-2-selective inhibitor, SC-558) bind to the same active site. (2) It is therefore possible to assume that a protein has an identical or nearly identical site of binding even if small molecules are different in structure. (3) Such a site comprises a pocket or pockets for docking these molecules and can correspondingly comprise a single module or a composite of several different modules. The crystallographic studies of Kurumbail, R. G., et al. (loc. cit.) and others on complexes of Cox-1 and Cox-2 with NSAIDs and SC-558 suggest the presence of such a composite of several different modules. (4) It is tempting to assume that, when several small molecules, despite their apparent difference in structure, are found to bind to the same protein with high affinity, they bind to the same or nearly identical site (converse of (2) above), except in cases where non-specific binding prevails such as due to van der Waals contact and/or electrostatic interaction. (5) It is also tempting to assume that such common site of binding for different small molecules is mostly in the form of a pocket, a seemingly unstable thermodynamic structure, and comprises a single module or a composite of several modules that have been evolutionally conserved. (6) When one finds that a set of small molecules bind to a definitive set of different proteins with high affinity, it can be an indication that these proteins have in common the same or nearly identical site for binding of those small molecules. (7) Comparison of amino acid sequences of these proteins may then be able to identify common or similar partial sequences and residues, as in the case of interactions of a single chemical compound with multiple proteins described previously. (8) It is possible that these common or similar partial sequences and residues as well as a chain or chains comprising them constitute a single module or a composite of several modules that have been evolutionally conserved. It is also possible that such modules form a pocket that is suitable for docking small molecules. (9) Cross-species comparison of sequences of evolutionally related proteins having high affinity for the same set of small molecules will further give assurance to the inference of an evolutionally conserved module and possibly of a pocket. (10) A significant difference in the intensity of affinity for a chemical compound in molecular association with related proteins such as Cox-1 and Cox-2 suggests the presence or absence of specific module(s) and corresponding pocket(s) in either of proteins (see, for example, Kurumbail, R. G., et al., loc. cit., for the presence of a SC-558-specific pocket of Cox-2). (In literature there is no clear distinction as to the size of a pocket. It is possible that a pocket of larger size comprises several pockets of smaller sizes. Such distinction is implicit in the above discussion.)

Databases resulting from the use of methods described above are readily constructed. Similarly constructed are user-interfaces that display, in tabulated and/or graphical format, the output resulting from the use of methods described above and/or the use of the databases constructed by the use of these methods.

Finally, it is necessary to devise a method of data mining to extract the relationship between (a) interactions of proteins or portions of proteins with chemical compounds and (b) interactions of the proteins or portions of proteins with other proteins or portions of proteins. This is accomplished by comparing profiles of interactions of proteins or portions of proteins with chemical compounds and profiles of interactions of those proteins or portions of proteins with other proteins or other portions of proteins that are recorded in databases and user-interfaces mentioned above. Databases and user-interfaces are constructed accordingly.

Software that enables all of the above can be readily written with the use of available knowledge and expertise. Media such as floppy disks, CDs, CD-ROMs, and MDs recording above-mentioned databases, user-interfaces and software are readily prepared with the use of available technology. Services relevant to the use of above-mentioned databases, user-interfaces, software and media can be readily provided.

It is emphasized that the first merit of this invention is in its ability to secure a promising pool of proteins as drug target. Also emphasized, as an even more important merit of this invention, is that, because the originator chemical compound is known, it provides an efficient method to discover and prepare new and valuable drugs directly through optimization of the originator. The principle of this invention applies to other fields of industry such as in agrochemical, food, environmental, fermentation, and veterinary industries where the interaction between chemical compound and protein is the subject of interest.

The technology for drug discovery as disclosed in this invention may be termed "chemo-proteomics" or "reverse proteomics." This is an approach that reverses the one-way upstream-to-downstream genomics/proteomics approach. It begins with the end (chemical compounds) and goes upward to the genome.

Any patents, patent applications, and publications cited herein are incorporated by reference.

### Brief Description of the Drawings.

Figure 1. Upon binding of the protein on the left hand side to that of the right hand side (a protein-protein interaction) the latter protein produces a morphological change (nose and jaw-like protrusions on the back of the head-like structure). This morphological (conformational) change may cause an effect, or it may lead to another set of protein-protein interaction.
Figure 2. The morphological change in the protein on the right hand side is inhibited from occurring when two different small molecules each having a different site of attachment are used in combination.
Figure 3. The motion of a protein is restricted by the presence of a small molecule in the movable structure of the protein. The function of the protein may be inhibited by this kind of restricted movability. Examples in this figure show a small molecule acting as a wedge inserted into a hinge-like or joint-like structure of the protein molecule.
Figure 4. Examples of cooperative small molecule-protein interactions. While this figure shows cooperative interactions produced by the same molecular species of chemical compound, a combination of different molecular species can produce a similar type of interactions, sometimes more effective ones.

The present invention is further illustrated by, though in no way limited to, the following examples.

### Example 1. Chemical-attached solid support, its use in separation of proteins and discovery and generation of a new drug.

A chemical compound of interest (originator) is attached, preferably by covalent bond, by use of an appropriate reaction and/or an appropriate spacer/linker substance (abbreviated to spacer hereafter) to a solid support such as beads. Various kinds of solid supports ready for use to couple small molecules in chemical reactions are commercially available such as from Pharmacia (for example, CNBr-activated Sepharose, activated thiol Sepharose, etc. where the size of spacer ranges from 0 to 12 atoms). The solid support is washed with appropriate solutions to remove extraneous substances, including the chemical compound and reagents having failed to react, and is loaded into an appropriate chromatographic column using an appropriate solvent. A mixture of proteins, which can contain unknown proteins, is dissolved in an appropriate aqueous solution and is added to the chromatographic column. Washing of the column is conducted with the use of an appropriate aqueous solvent so that those proteins that do not have sufficient affinity for the chemical compound are washed away. Elution is achieved by using a solution containing the chemical compound of interest that is originally linked to the solid support but is in free form. Free form of the compound will compete for binding to the proteins bound to the solid support and will free them from it. Additionally an appropriate aqueous solvent having a particular range respectively in terms of pH and ionic strength may be employed. Elution can also be done in a stepwise fashion using solutions of the compound at graded concentrations and/or solvents of graded pH and ionic strength. The eluate is fractionally collected and concentrated by the use, for example, of a micro-filter. Each fraction is adjusted appropriately in terms of protein concentration and is submitted to gel electrophoresis. Proteins on the gel are visualized by staining, for example, with Coomassie Blue. Each band is compared with the standard molecular weight marker bands, eluted and submitted to amino acid sequence analysis. Based directly on the data of amino acid sequence of each protein or based indirectly on the cDNA sequence data which are obtained by designing appropriate nucleic acid probes from the amino acid sequence data, obtaining from appropriate cDNA libraries a cDNA molecule hybridizing to the probes, and sequencing the cDNA molecule, the databases such as of NCBI or EMBL are searched for information about the protein. If the protein is found to be an interesting drug target, then the process of optimization is initiated to obtain a compound with higher affinity and specificity based on the structure of the originator. The process of optimization can also be guided by other appropriate assays than affinity as previously described. Such optimization of the originator is expected to lead to discovery and generation of a new and valuable drug. If database searches fail to identify the protein, the data are stored and, when additional information becomes available, the protein is re-evaluated as to whether it is a likely drug target. It is possible to obtain proteins of desired affinity for a chemical compound by appropriately adjusting pH and ionic strength of washing solvent. For example, the lower the ionic strength, the more proteins with lower affinity for the chemical compound are expected to remain in the chromatographic column. The ionic strength can be high so as to effect complete elution of bound proteins but, if desired, it can be graded to effect graded elution of proteins according to affinity.

As long as a chemical compound attached to solid support is used as bait, so to speak, for proteins, any modification is feasible. For example, the solid support can be in the form of plate. Protein solution can flow over the chemical compound-attached plate, or the plate can be immersed in protein solution, and, after washing of the plate, proteins of desired affinity can be eluted out from the plate. The plate can also be in the form of a well.

When elution is accomplished by solutions of chemical compounds that are the same as those attached to solid support, a mixture of beads carrying different chemical compounds can be packed into a chromatographic column. For example, beads carrying compounds, A, B, and C are mixed or prepared, and packed into one column. A mixture of proteins is then applied to the column, washed, and eluted first with a solution containing A, second with a solution containing B, and then with a solution containing C. The first eluate is expected to contain proteins having affinity for compound A, the second those for compound B and the last those for compound C. This mode of elution of proteins is termed "differential elution by stepwise application of solutions containing different chemical compounds in free form." This situation is applicable to other forms of solid support, i.e., plate and well where simultaneously different chemical compounds are attached.

### Example 2. A multiplexed system comprising chemical-attached solid support and its use in separation of proteins.

A plate with multiples of wells, for example of 96 wells, can accommodate multiples of different chemical compounds. A solution containing a mixture of proteins is made in contact with such plate at once and, after washing of the plate with washing solvent, elution is effected separately from well to well. This can be done conveniently by automatic filling of the wells with eluting solvent and, after standing for a while for binding to take place between proteins and the chemical compound, by automatic sucking of the content of each well. To collect eluate from each well, alternatively, a pore is made in each well so that eluate drops into each of separated receiver wells due to gravity. With the additional use of pins, drops are guided into each of receiver well more efficiently. Solvents for washing and elution can be made different from well to well manually but more conveniently by automation through prior computer programming of filling device.

Another version is a plate consisting of multiplexed mini-chromatographic columns. A plate of certain thickness is cut out to make multiples of pores. The bottom surface of the plate is tightly covered with a sheet of material that can simultaneously act as a filter to pass the solvent and as a support to retain the chemical compound-attached solid material. Each of the pores is loaded with chemical compound-attached solid support that differs in terms of attached chemical compound. Again a solution containing a mixture of proteins is made in contact with the chemical compound-attached solid support from over the plate and washing and elution is effected, at once with all of the pores, or separately from pore to pore.

### Example 3. A method and a device using solid support to capture proteins present on cell surface.

To a solid support in the form of beads, plate, or wells is attached a chemical compound according to the method illustrated in Example 1, and cells are captured on to the solid support in a single substance version of Example 1 or in a multiplexed version of Example 2. Antibodies to known cell surface proteins are employed to distinguish between different cell surface proteins bound to the chemical compound. In practice, such a cell carrying on its surface a protein reacting to the employed antibody will be released from the solid support, demonstrating in the end what cell surface protein possesses affinity for the chemical compound. Cells can be sorted prior to the operation with respect to class, origin and function. This preparatory procedure reduces the degree of uncertainty in terms of the results obtained. In order to efficiently conduct protein identification, for example, a dichotomized mixture of antibodies is used as the first test, either of the two mixtures which has proven to be positive is then subdivided (actually previously prepared), and this process is repeated until a single antigenic protein becomes identified. Other manner of division than dichotomy can also be employed. A reservation is that the antibody is not almighty and that the cell bound to the chemical compound through a protein may not be freed by the corresponding antibody because of possible difference in the site or mode (for example, electrostatic and other) of binding to the protein by the chemical compound and the antibody.

### Example 4. Use of cells that have been genetically engineered to express on their surface a specific protein in an enriched quantity.

A known protein is expressed on the surface of a cell in an enriched quantity. These cells are applied to the multiplexed chemical-attached solid support of Example 3 to examine which chemical compound has affinity for the cells. Alternatively, a cell panel consisting of cells differentially expressing proteins is prepared and applied to chemical compound-attached solid support of Example 3. Differentiation of cell surface-expressed proteins is effected by use of antibodies as illustrated in Example 3.

### Example 5. Use of sorted protein mixtures.

According to literature, it is practically possible to obtain a collection of proteins (i.e., protein library) sorted with respect to class, subcellular localization and function. For example, cDNA molecules encoding secretable and cell surface proteins are collectively obtained by the method of Honjo et al. (US Patent 5,525,486), of Jacobs (US Patent 5,536,637) and of Tuchiya et al. (WO99/60113). These cDNA molecules, if not of full-length, after adding appropriate procedures to obtain full-length cDNA, are used to obtain a library of secretable and cell surface proteins. Similarly, a library of proteins capable of migrating into the cell nucleus is prepared from cDNA molecules obtained by the method of Ueki and Yano (Tokukai 2000-50882, a publication of Japanese patent application). Already many GPCR protein-encoding cDNA molecules have been isolated according to literature regardless of whether their function and/or ligands are known. Such cDNA molecules are used to prepare a GPCR protein library. It is also possible to prepare a library of phosphorylated proteins, notably that of kinases, by biotinylating them with maleinimidated biotin and affinity separation of biotinylated molecules with an avidin column. There are many proteins that are known to participate in inflammatory reactions, including cytokines and interleukins. These can be used to prepare a library of inflammatory proteins.

### Example 6. Methods for obtaining membrane-associated proteins in the form of extracellular virions.

Certain viruses, when genetically engineered, express membrane-associated proteins of different organisms that maintain their original function. An example is the use of Spodoptera frugiperda (Sf9) cells infected with recombinant baculovirus (Autographa californica multiple nuclear polyhedrosis virus) (Bouvier, M., et al. PCT WO 98/46777; Loisel, T.P., et al. Nature Biotechnology (1997) 15:1300-1304). These researchers found that virus particles released from Sf9 cells infected with recombinant baculovirus coding for the human beta 2-adrenergic receptor cDNA contained corresponding glycosylated and biologically active receptor. They also showed that virus particles derived from cells infected with baculovirus encoding M1-muscarinic or D1-dopaminergic receptors contained respective receptors. They further comment that harvesting extracellular virions from Sf9 cells infected with GPCR-encoding baculoviruses may be an easy and generally applicable method to produce large amounts of biologically active receptors and that this method may represent an advantageous alternative to such purification schemes as using crude Sf9 membrane preparations that require an affinity chromatography step to eliminate the inactive (misfolded) forms of the receptor (Bouvier, M., et al. Current Opinion in Biotechnology (1998) 9:522-527). A virus-cell system may be present that is capable of expressing biologically active exogenous membrane proteins that originally reside intracellularly such as associated with endoplasmic reticulum, nuclear membrane and Golgi apparatus.

### Example 7, Use of the BIACORE method and the like.

One of more sophisticated methods of solid support-assisted affinity evaluation is achieved by the use of surface plasmon resonance measurement, notably as commercialized by BIACORE International AB, that can yield quantitative data for affinity readily. Devices similar to that of BIACORE capable of yielding quantitative information can also be utilized. In this scheme either chemical compound (mainly small molecule) or protein is attached to solid support.

### Example 8. Methods of affinity evaluation without requiring chemical modification of compounds.

Solid support-assisted affinity evaluation requires chemical modification of small molecule compounds to attach them to solid support. Such chemical modification is not always easy. To circumvent this, methods not requiring chemical modification can be used. One of the methods is size fractionation by the use of gel filtration, ultrafiltration or dialysis. A method of evaluating the interaction between a protein or a portion of a protein and a chemical compound consists of the following sequential steps:
(1)A chemical compound to be evaluated is mixed with a library containing proteins and/or portions of proteins and, after allowing some time for interaction to occur, resulting mixture is subjected to gel filtration or ultrafiltration under a condition where dissociation of the chemical compound with proteins or portions of proteins in the library is avoided.
(2) Step (1) is repeated until most of proteins or portions of proteins in the library are separated into fractions whereby each of the fractions contains a single species of protein or a single species of portion of a protein.
(3) Each fraction resulting from Steps (1) and (2) that contains a single species of protein or a single species of portion of a protein is then subjected to a condition that effectively liberates the chemical compound from proteins or portions of proteins in the library and is further subjected to gel filtration, ultrafiltration, or dialysis.
(4) Each fraction resulting from Step (3) is examined for the presence or absence of said chemical compound. If present, said chemical compound is concluded to bind to the single species of protein or portion of a protein.
(5) Sum of the amounts of the chemical compound resulting from Step (4) is converted to original concentration in corresponding fraction resulting from Step (3). This original concentration and the concentration of corresponding single species of protein or portion of a protein in each of fractions resulting from Step (3) give quantitative information on the intensity of affinity of the chemical compound for the single species of protein or portion of a protein.

To avoid dissociation of the chemical compound with proteins or portions of proteins a physiological condition or a condition close to it is preferred. A condition that effectively liberates the compound from the protein is achieved by the adjustment of pH, the application of high ionic strength and the use of water-miscible organic solvents such as glycols, methanol, ethanol, propanol, acetonitrile, dimethyl sulfoxide, tetrahydrofuran, and trifluoroacetic acid, used either singly or in a combined manner. As gel filtration (size exclusion chromatography) excludes proteins earlier and because ultrafiltration filtrates small molecules earlier, the use of the former in Steps (1) and (2) and the use of the latter in Step (3) after small molecule liberation may be preferable if the two technologies are used. Liberated compound can be conveniently monitored by UV spectrophotometry or other available means for detection or quantification. If a means to differentially detect or quantify each of several compounds is available, it is possible to cause interactions between a mixture of those compounds and the library of proteins, i.e., in mixture-versus-mixture mode.

### Example 9. Use of proteins attached to solid support.

Instead of attaching chemical compounds to a solid support, it is possible to attach proteins to it to study compound-protein interactions. For example, the systems illustrated in Example 2 can be used under this scheme. After washing the wells or mini-chromatographic columns, a compound-liberating condition is applied and liberation of the compound being evaluated is examined with respect to each of the wells or mini-chromatographic columns. So-called protein chips may be fitted to this kind of use. The use of the BIACORE method or the like under this protein-to-solid support scheme is advantageous as it does not require the step of liberating compounds, as described in Example 7.

### Example 10. Methods to assess if chemical compound-protein interaction is biologically significant.

For purpose of explanation, chemical compound and protein involved in the interaction are called the chemical compound and the protein, respectively.

It is recommended that cells of many different kinds (including cell lines) are ready for use. These cells (test cells) can be of yeast, C. elegans, drosophila and other animals (for environmental and agrochemical purposes, microorganisms and plants) including mammals and, above all, humans. Recommended to be ready also for use as test cells are those known to demonstrate morphological, physicochemical and/or biochemical characteristics including secretion of characteristic small molecule ligands, peptides and proteins. It is further advantageous to be ready with means to monitor changes in intracellular as well as extracellular parameters. Examples of such physicochemical and/or biochemical parameters include pH, calcium, cyclic AMP and cyclic GMP concentrations. Optical and electrophysiological changes may also be monitored. The first thing that can be performed even without the knowledge of what class the protein belongs to is to see what happens in the expression profile of a test cell treated with the chemical compound of sub-toxic concentration at the mRNA level in comparison with what happens in the absence of treatment with it (control). If some difference is observed, it does not necessarily mean that the difference is due to the interaction being evaluated, unless there is significantly high affinity and specificity of the compound for the protein and unless a reasonably low concentration has been employed for the compound in the expression profiling. To clarify this, an antisense molecule (AS) corresponding to the protein being evaluated is used in place of the chemical compound. If the AS produces a change in expression profile that is either similar or opposite in direction to the change produced by the treatment of the cell with the chemical compound, it is concluded, as described elsewhere with respect to agonist and antagonist, that the interaction is biologically significant. While technically laborious, knock-out cells lacking the expression of the evaluated protein and cells that over-express it may be additionally useful. These cells are used to see if the biological change that is produced by the chemical compound in the corresponding normal cells is similar or opposite in direction to the change produced either of these genetically engineered cells. The classification or identification of the protein through database search with the use of sequence information is quite helpful. According to the class of proteins the following evaluation is carried out:
1. Enzymes (including kinases). Devise or use a method to assess the enzyme activity and compare the activity in the presence or absence of the chemical compound being evaluated.
2. Secreted proteins. If the function of the evaluated protein is known, appropriate assay methods are devised to see if that function is affected by the presence of the evaluated chemical compound. If it is unknown, it is necessary to find what happens in test cells in the presence of the evaluated protein with respect to their morphology, physicochemistry (such as pH), biochemistry, electrophysiology, or molecular biology (such as expression profiles at the mRNA level). Once a change is identified, assessment is made as to if such change is affected by the presence of the evaluated compound. In addition, the methods described below for proteins associated with cell surface membrane can be used.
3. Proteins associated with cell surface membrane. Compare expression profiles at the mRNA level of test cells in the presence or absence of the evaluated compound. With significantly high affinity and specificity of the compound for the cell membrane-associated protein and with a reasonably low concentration employed for the compound, it can be preliminarily inferred that a change in the expression profile, when observed, is a result of assumed interaction between the compound and the protein and that such interaction is biologically significant. To further ascertain this inference it is necessary to compare the expression profiles in the presence of the compound and in the presence of AS corresponding to the protein in place of the compound. If the interaction is significant, AS is expected to produce a similar expression profile or an inverse of it. If a protein similar in sequence to the protein being evaluated is known and further if agonist(s) and/or antagonist(s) to that protein is/are known, an experiment is performed to see if the presence of the compound and the presence of at least one of such substances demonstrate changes of similar or opposite direction in any of cell-free and cell-based test systems. Observation of such changes is a positive sign for the biological significance of the interaction.
4. Nuclear receptors. Methods identical to those described for proteins associated with cell surface membrane are used.
5. Intracellular signaling proteins. Methods identical to those described for proteins associated with cell surface membrane are used.
6. Transcription factors and proteins related to transcription. Methods identical to those described for proteins associated with cell surface membrane are used.
7. Other proteins including unclassified or unidentified proteins. Some of the methods described for proteins associated with cell surface membrane are used.

### Example 11. Other methods of detecting or quantifying the interaction between a chemical compound and a protein.

Further examples of detecting or quantifying the interaction between a chemical compound and a protein include determination of the change in resonant frequency of quartz oscillator, determination of the change in surface elastic wave, and use of mass spectroscopy.

### Example 12. Use of capillary electrophoresis in separation of proteins.

As proteins associated with any chemical compound have, in general, mobilities that are different from corresponding proteins in non-associated (i.e., free) form, it is possible to separate, detect or quantify proteins in associated form from free counterparts. This method can be used to study the interaction between a chemical compound and a protein or a portion of a protein.

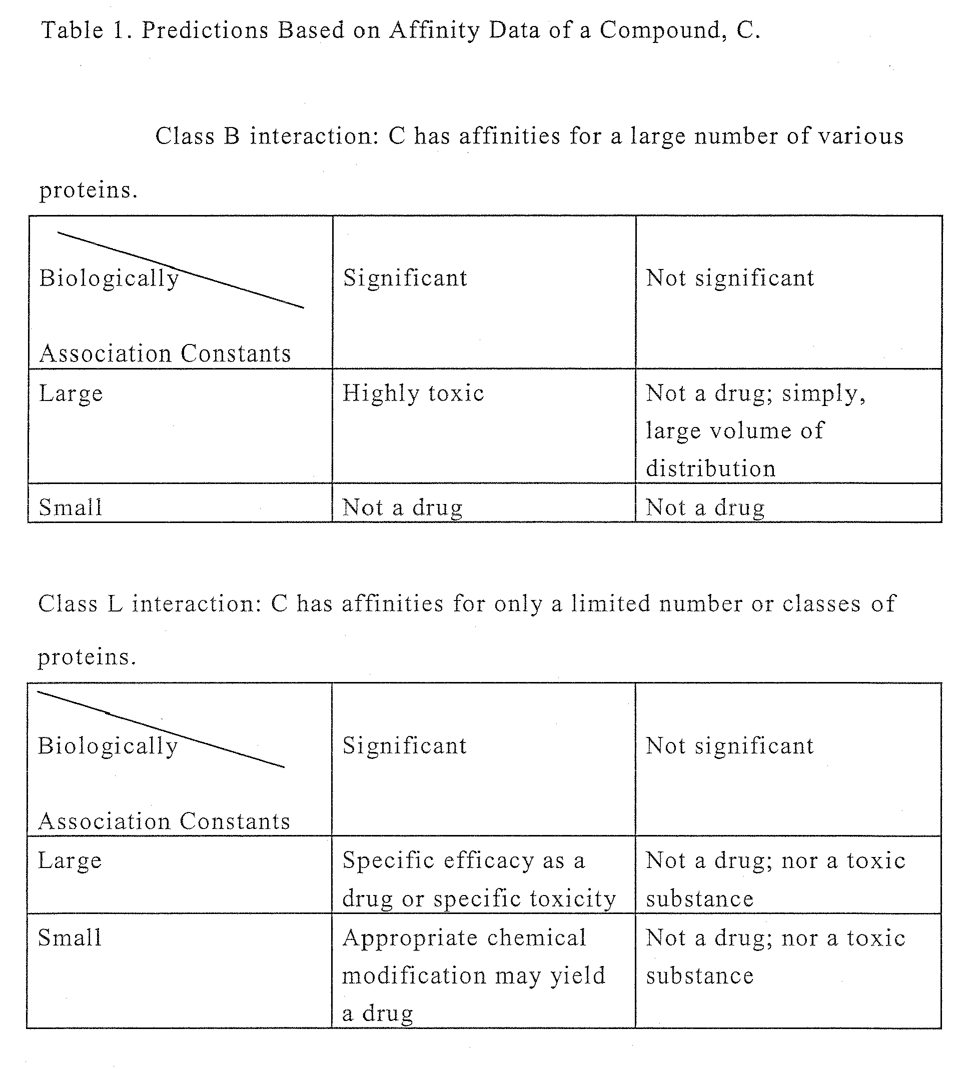

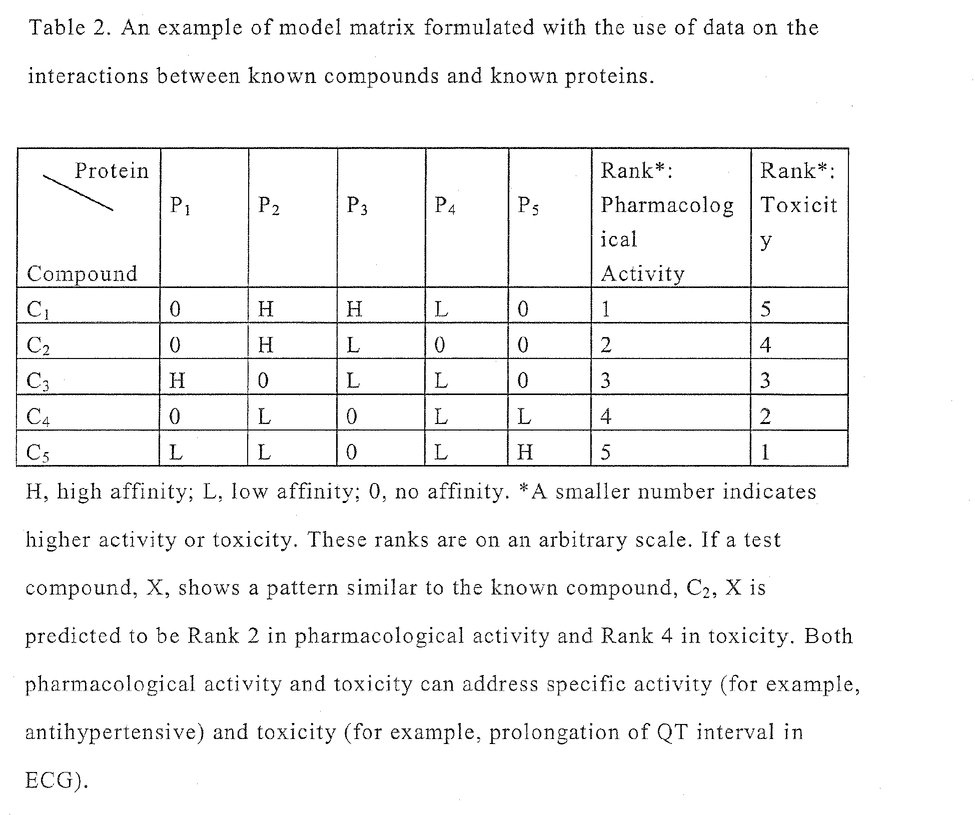

### Items

1. A collection of data, database, or catalog concerning the interaction between a protein or a portion of a protein and a chemical compound.
2. A collection of data, database, or catalog according to item 1, which is characterized by tabulated description of interaction between a protein or a portion of a protein and a chemical compound.
3. A collection of data, database, or catalog according to item 1 or item 2, wherein said chemical compound is selected from a population consisting of chemical compounds of less than 1,600 in molecular weight.
4. A collection of data, database, or catalog according to item 1 or item 2, wherein said chemical compound is selected from a population consisting of chemical compounds of less than 1,000 in molecular weight.
5. A collection of data, database, or catalog according to item 1 or item 2, wherein said chemical compound is selected from a population consisting of chemical compounds of less than 600 in molecular weight.
6. A collection of data, database, or catalog according to item 1 or item 2, wherein said chemical compound is selected from a population consisting of chemical compounds of less than 500 in molecular weight.
7. A collection of data, database, or catalog according to any of items 1 through 6, wherein said chemical compound is selected from a population of drugs approved for medical use.
8. A collection of data, database, or catalog according to any of items 1 through 7, wherein description of presence or absence of said interaction is included.
9. A collection of data, database, or catalog according to any of items 1 through 8, wherein said interaction is defined by a parameter for intensity of affinity and/or by mode of interaction and/or by structural element of interaction.
10. A collection of data, database, or catalog according to item 9, wherein said parameter for intensity of affinity means (a) an association rate constant and/or a dissociation rate constant, and/or (b) an equilibrium constant of association and/or an equilibrium constant of dissociation.
11. A collection of data, database, or catalog according to any of items 9 and 10, wherein said mode of interaction means any or any combination of an interaction due to van der Waals force, hydrogen bonding, electrostatic interaction, charge transfer, hydrophobic, hydrophilic and lipophilic interactions, and cooperative binding or cooperative interaction.
12. A collection of data, database, or catalog according to any of items 9 through 11, wherein said structural element of interaction means any or any combination of site of interaction, structure of said site of interaction, interacting group, interacting amino acid residue, interacting atom, interacting surface, and relative position, in 1-, 2-, or 3-dimensional space, of interacting group, interacting amino acid residue, interacting atom and/or interacting surface.
13.A collection of data, database, or catalog concerning the interaction between a protein or a portion of a protein and each of a multitude of chemical compounds.
14.A collection of data, database, or catalog according to item 13, which is characterized by tabulated description of interaction between a protein or a portion of a protein and each of a multitude of chemical compounds.
15. A collection of data, database, or catalog concerning the interaction between each of a multitude of proteins or portions of said proteins and a chemical compound.
16. A collection of data, database, or catalog according to item 15, which is characterized by tabulated description of interaction between each of a multitude of proteins or portions of said proteins and a chemical compound.
17. A collection of data, database, or catalog according to any of items 13 through 16, wherein said chemical compound is as defined in any of items 3 through 6.
18. A collection of data, database, or catalog according to item 17, wherein said chemical compound is as defined in item 7.
19. A collection of data, database, or catalog according to any of items 13 through 18, wherein description of presence or absence of said interaction is included.
20. A collection of data, database, or catalog according to any of items 13 through 19, wherein said interaction is defined by a parameter for intensity of affinity and/or by mode of interaction and/or by structural element of interaction.
21.A collection of data, database, or catalog according to item 20, wherein said parameter for intensity of affinity means (a) an association rate constant and/or a dissociation rate constant, and/or (b) an equilibrium constant of association and/or an equilibrium constant of dissociation.
22. A collection of data, database, or catalog according to any of items 20 and 21, wherein said mode of interaction means any or any combination of an interaction due to van der Waals force, hydrogen bonding, electrostatic interaction, charge transfer, hydrophobic, hydrophilic and lipophilic interactions, and cooperative binding or cooperative interaction.
23.A collection of data, database, or catalog according to any of items 20 through 22, wherein said structural element of interaction means any or any combination of site of interaction, structure of said site of interaction, interacting group, interacting amino acid residue, interacting atom, interacting surface, and relative position, in 1-, 2-, or 3-dimensional space, of interacting group, interacting amino acid residue, interacting atom and/or interacting surface.
24.A collection of data, database, or catalog according to any of items 1 through 23, wherein said protein or said portion of a protein is derived from cell lysate.
25. A collection of data, database, or catalog according to any of items 1 through 24, wherein said protein or said portion of a protein is prepared artificially by genetic engineering.
26. A collection of data, database, or catalog according to any of items 1 through 25, wherein said protein or said portion of a protein is expressed from full-length cDNA.
27.A collection of data, database, or catalog according to any of items 1 through 26, wherein said protein or said portion of a protein is focused with respect to class, activity, or localization.
28. A collection of data, database, or catalog according to item 27, wherein said activity is enzymatic.
29.A collection of data, database, or catalog according to item 27, wherein said localization is either cell surface, cytoplasm or nucleus.
30.A collection of data, database, or catalog according to item 27, wherein said localization is cell type, tissue origin, and/or organ origin.
31. A collection of data, database, or catalog according to any of items 1 through 26, wherein said protein or said portion of a protein is associated with a membranous structure of a cell.
32. A collection of data, database, or catalog according to item 31, wherein said protein or said portion of a protein is a GPCR or is derived thereof.
33.A collection of data, database, or catalog according to any of item 31 and item 32, wherein said protein or said portion of a protein is expressed in extracellular virions.
34. A collection of data, database, or catalog according to any of items 31 through 33, wherein said protein or said portion of a protein is obtained physico-chemically by treatment of cells with a solution containing a mild detergent or a mixture of mild detergent.
35. Method of evaluating the interaction between a protein or a portion of a protein and a chemical compound characterized by said chemical compound being selected from a population consisting of chemical compounds of less than 1,600 in molecular weight.
36. Method of evaluating the interaction between a protein or a portion of a protein and a chemical compound characterized by said chemical compound being selected from a population consisting of chemical compounds of less than 1,000 in molecular weight.
37. Method of evaluating the interaction between a protein or a portion of a protein and a chemical compound characterized by said chemical compound being selected from a population consisting of chemical compounds of less than 600 in molecular weight.
38.Method of evaluating the interaction between a protein or a portion of a protein and a chemical compound characterized by said chemical compound being selected from a population consisting of chemical compounds of less than 500 in molecular weight.
39.Method of evaluating the interaction between a protein or a portion of a protein and a chemical compound according to any of items 35 through 38 characterized by said chemical compound being selected from a population of drugs approved for medical use.
40. Method of evaluating the interaction between a protein or a portion of a protein and a chemical compound characterized by said protein or said portion of a protein being derived from cell lysate.
41.Method of evaluating the interaction between a protein or a portion of a protein and a chemical compound characterized by said protein or said portion of a protein being prepared artificially by genetic engineering.
42. Method of evaluating the interaction between a protein or a portion of a protein and a chemical compound characterized by said protein or said portion of a protein being expressed from full-length cDNA.
43. Method of evaluating the interaction between a protein or a portion of a protein and a chemical compound according to any of items 40 through 42 characterized by said protein or said portion of a protein being focused with respect to class, activity, or localization.
44. Method of evaluating the interaction between a protein or a portion of a protein and a chemical compound according to item 43 characterized by said activity being enzymatic.
45.Method of evaluating the interaction between a protein or a portion of a protein and a chemical compound according to item 43 characterized by said localization being either cell surface, cytoplasm or nucleus.
46. Method of evaluating the interaction between a protein or a portion of a protein and a chemical compound according to item 43 characterized by said localization being cell type, tissue origin, and/or organ origin.
47. Method of evaluating the interaction between a protein or a portion of a protein and a chemical compound according to any of items 40 through 42 characterized by said protein or said portion of a protein being associated with a membranous structure of a cell.
48.Method of evaluating the interaction between a protein or a portion of a protein and a chemical compound according to item 47 characterized by said protein or said portion of a protein being a GPCR or being derived thereof.
49. Method of evaluating the interaction between a protein or a portion of a protein and a chemical compound characterized by the carrier of said protein or said portion of a protein being a cell.
50. Method of evaluating the interaction between a protein or a portion of a protein and a chemical compound characterized by the carrier of said protein or said portion of a protein being extracellular virions.
51. Method of evaluating the interaction between a protein or a portion of a protein and a chemical compound characterized by said protein or said portion of a protein being obtained physico-chemically by treatment of cells with a solution containing a mild detergent or a mixture of mild detergent.
52. Method of evaluating the interaction between a protein or a portion of a protein and a chemical compound according to any of items 35 through 51, wherein said interaction is defined by a parameter for intensity of affinity and/or by mode of interaction and/or by structural element of interaction.
53. Method of evaluating the interaction between a protein or a portion of a protein and a chemical compound according to item 52, wherein said parameter for intensity of affinity means (a) an association rate constant and/or a dissociation rate constant, and/or (b) an equilibrium constant of association and/or an equilibrium constant of dissociation.
54. Method of evaluating the interaction between a protein or a portion of a protein and a chemical compound according to any of items 52 and 53, wherein said mode of interaction means any or any combination of an interaction due to van der Waals force, hydrogen bonding, electrostatic interaction, charge transfer, hydrophobic, hydrophilic and lipophilic interactions, and cooperative binding or cooperative interaction.
55.Method of evaluating the interaction between a protein or a portion of a protein and a chemical compound according to any of items 52 through 54, wherein said structural element of interaction means any or any combination of site of interaction, structure of said site of interaction, interacting group, interacting amino acid residue, interacting atom, interacting surface, and relative position, in 1-, 2-, or 3-dimensional space, of interacting group, interacting amino acid residue, interacting atom and/or interacting surface.
56. Method of identifying a protein or a portion of a protein eligible as a new drug target, comprising:
   (1)selecting proteins or portions of proteins of desired affinity and specificity for a selected target compound,
   (2)characterizing said proteins or said portions of proteins with respect to structure and function, and
   (3)ehoosing a protein or a portion of protein of desired function.
57. Method of discovering a drug, comprising:
   (1)examining the chemical structure of said selected target compound employed in the use of the method claimed in item 56, and
   (2)chemically modifying the structure of said selected target compound to optimize affinity and specificity of modified compound for said protein or said portion of a protein eligible as new drug target according to item 56.
58.Method of identifying a protein or a portion of a protein eligible as a new drug target according to item 56, wherein the molecular weight of said selected target compound is less than 1,600.
59. Method of identifying a protein or a portion of a protein eligible as a new drug target according to item 56, wherein the molecular weight of said selected target compound is less than 1,000.
60.Method of identifying a protein or a portion of a protein eligible as a new drug target according to item 56, wherein the molecular weight of said selected target compound is less than 600.
61. Method of identifying a protein or a portion of a protein eligible as a new drug target according to item 56, wherein the molecular weight of said selected target compound is less than 500.
62.Method of identifying a protein or a portion of a protein eligible as a new drug target according to item 56, wherein said selected target compound is approved for medical use.
63.Method of identifying a protein or a portion of a protein eligible as a new drug target according to any of items 58 through 61, wherein said selected target compound is approved for medical use.
64. Method of discovering a drug according to item 57, wherein the molecular weight of said selected target compound is less than 1,600.
65. Method of discovering a drug according to item 57, wherein the molecular weight of said selected target compound is less than 1,000.
66. Method of discovering a drug according to item 57, wherein the molecular weight of said selected target compound is less than 600.
67.Method of discovering a drug according to item 57, wherein the molecular weight of said selected target compound is less than 500.
68. Method of discovering a drug according to item 57, wherein said selected target compound is approved for medical use.
69. Method of discovering a drug according to any of items 64 through 67, wherein said selected target compound is approved for medical use.
70. A collection of data, database, or catalog according to any of items 1 through 34, wherein said protein or said portion of a protein being of microorganism, plant, animal, insect, mammal, or human origin.
71. Method of evaluating the interaction between a protein or a portion of a protein and a chemical compound according to any of items 35 through 55, wherein said protein or said portion of a protein being of microorganism, plant, animal, insect, mammal, or human origin.
72. Method of identifying a protein or a portion of a protein eligible as a new drug target according to any of item 56 and items 58 through 63, wherein said protein or said portion of a protein being of microorganism, plant, animal, insect, mammal, or human origin.
73. Method of discovering a drug according to item 57 and items 64 through 69, wherein said protein or said portion of a protein being of microorganism, plant, animal, insect, mammal, or human origin.
74.A collection of data, database, or catalog according to any of items 1 through 34 and item 70, wherein said chemical compound is obtained during drug discovery research.
75.Method of evaluating the interaction between a protein or a portion of a protein and a chemical compound according to any of items 35 through 55, wherein said chemical compound is obtained during drug discovery research.
76. Method of identifying a protein or a portion of a protein eligible as a new drug target according to any of item 56 and items 58 through 63, wherein said selected target compound is obtained during drug discovery research.
77. Method of discovering a drug according to any of item 57 and items 64 through 69, wherein said selected target compound is obtained during drug discovery research.
78.Method of identifying a protein or a portion of a protein responsible for toxicity or an adverse reaction of a chemical compound, comprising:
   (1)selecting proteins or portions of said proteins with high affinity and specificity for said chemical compound,
   (2)characterizing said proteins or portions of said proteins with respect to structure and function, and
   (3)choosing a protein or a portion of a protein responsible for toxicity or said adverse reaction of said chemical compound.
79. Method of discovering a chemical compound with reduced degree of toxicity and adverse reaction, comprising:
   (1)examining the chemical structure of said chemical compound employed in the use of the method claimed in item 78, and
   (2)chemically modifying the structure of said chemical compound to minimize affinity of modified compound for said protein responsible for toxicity or adverse reaction.
80. Method of identifying a protein or a portion of a protein responsible for toxicity or an adverse reaction of a chemical compound according to item 78, wherein said chemical compound is obtained during drug discovery research or is environmentally hazardous.
81. Method of discovering a chemical compound with reduced degree of toxicity and adverse reaction according to item 79, wherein said chemical compound is obtained during drug discovery research or is environmentally hazardous.
82.A collection of data, database, or catalog according to any of items 1 through 34 and item 70, wherein said chemical compound or compounds being environmentally hazardous.
83. Method of listing drug-like compounds characterized by (a) examination of the chemical structure of said selected target compound employed in the use of the method claimed in any of item 56 and items 58 through 63, and (b) virtual synthesis of drug-like compounds derivable from said selected target compound by the use of technology in computational chemical synthesis.
84.A collection of data, database, or catalog constructed by the use of the method claimed in item 83.
85. Method of modifying the activity of a protein or a portion of a protein characterized by the use of a chemical compound that acts as an obstacle to the movement of a movable structure of said protein or said portion of a protein.
86. Method of modifying the activity of a protein or a portion of a protein with a chemical compound that acts as a wedge inserted into a hinge-like or joint-like structure of said protein.
87.Method of modifying the activity of a protein or a portion of a protein characterized by the use of a combination of different chemical compounds that bind cooperatively to said protein or said portion of a protein.
88.Method of modifying a protein-protein interaction characterized by the use of a combination of different chemical compounds.
89.Method of modifying a protein-protein interaction according to item 88, wherein said chemical compounds bind to different sites of attachment on interacting surfaces of proteins.
90. Method of modifying a protein-protein interaction according to item 88, wherein at least one of said chemical compounds attaches to a site not situated on the interacting surface of either protein.
91.Method of modifying a protein-protein interaction according to item 88 characterized by the use of at least one of said chemical compounds that act as an obstacle to the movement of a movable structure of either protein.
92.Methods of modifying a protein-protein interaction according to item 88, wherein at least one of said chemical compounds acts as a wedge inserted into a hinge-like or joint-like structure of either protein.
93.Method of modifying a protein-protein interaction according to item 88, wherein said chemical compounds bind cooperatively to either or both of proteins.
94. Therapeutic use of the method claimed in any or any combination of items 85 through 93.
95.Therapeutic use of a chemical compound that acts as an obstacle to the movement of a movable structure of said protein or said portion of a protein to modify the activity of said protein.
96. Therapeutic use of a chemical compound that acts as a wedge inserted into a hinge-like or joint-like structure of a protein to modify the activity of said protein.
97. Therapeutic use of a combination of different chemical compounds that bind cooperatively to a protein to modify the activity of said protein.
98. Therapeutic use of a combination of different chemical compounds to modify a protein-protein interaction.
99. Therapeutic use of a combination of different chemical compounds according to item 98, wherein said chemical compounds bind to different sites of attachment on interacting surfaces of proteins.
100. Therapeutic use of a combination of different chemical compounds according to any of items 98 and 99, wherein at least one of said chemical compounds attaches to a site not situated on the interacting surface of either protein.
101. Therapeutic use of a combination of different chemical compounds according to any of items 98 through 100, wherein at least one of said chemical compounds acts as an obstacle to the movement of a movable structure of either protein.
102. Therapeutic use of a combination of different chemical compounds according to any of items 98 through 101, wherein at least one of said chemical compounds acts as a wedge inserted into a hinge-like or joint-like structure of either protein.
103. Therapeutic use of a combination of different chemical compounds according to any of items 98 through 102, wherein said chemical compounds bind cooperatively to either or both of proteins.
104. A collection of data, database, or catalog listing chemical compounds that commonly bind to a protein or a portion of a protein.
105. A collection of data, database or catalog listing chemical compounds that bind to either partner protein or a portion of either partner protein in a protein-protein interaction.
106. Method of evaluating the biological significance of an interaction between a chemical compound and a protein comprising:
   (1)comparing the expression profile at the mRNA level of a test cell treated with said chemical compound of reasonably low concentration with control expression profile when there is significantly high affinity and specificity of said compound for said protein, and/or
   (2)using an AS corresponding to said protein in place of said chemical compound to see if said AS produces a change in the expression profile that is either similar or opposite in direction to the change produced by the treatment of the cell with said chemical compound, and/or
   (3) using a knock-out cell lacking the expression of said protein or a cell over-expressing said protein to see if the biological change that is produced by said chemical compound in the corresponding normal cell is similar or opposite in direction to the change produced either of these genetically engineered cells, and/or
   (4)classifying or identifying said protein through database search with the use of sequence information, and/or
   (5)performing the following evaluation according to the class of said protein:
      1) Enzymes (including kinases). Devise or use a method to assess the enzyme activity and compare the activity in the presence or absence of saod chemical compound being evaluated.
      2) Secreted proteins. (a) If the function of said protein is known, appropriate assay methods are devised to see if that function is affected by the presence of said chemical compound. (b) If it is unknown, first find what happens in test cells in the presence of said protein with respect to their morphology, physicochemistry, biochemistry, optical change, or electrophysiology. Once a change is identified, then assess as to if such change is affected by the presence of said compound. In addition or alternatively, use the methods described for proteins associated with cell surface membrane.
      3) Proteins associated with cell surface membrane. If a protein similar in sequence to said protein being evaluated is known and further if an agonist or antagonist to that protein is known, an experiment is performed to see if the presence of said compound and the presence of agonist or antagonist demonstrate changes of similar or opposite direction in any of cell-free and cell-based test systems.
      4) Nuclear receptors, intracellular signaling proteins, transcription factors and proteins related to transcription. The method identical to that described for proteins associated with cell surface membrane is used.
107. Method of identifying a candidate for drug or toxic substance characterized by selecting a compound that has biologically significant affinities for a limited number or classes of proteins or portions of said proteins.
108. Method of discovering a drug or a non-toxic substitute for a toxic substance characterized respectively by optimizing or minimizing affinities for said proteins identified by the method claimed in item 107 by chemical modification of said candidate.
109. Method of defining pharmacology or toxicology of a chemical compound characterized by identification of functions of proteins with which said compound interacts in a biologically significant manner.
110. Method of predicting the pharmacological activity and toxicity of a test chemical compound characterized by comparing the affinity profile of said test chemical compound with a model matrix of affinity profiles that is formulated with the use of data on the interactions between known compounds and known proteins.
111. Method of identifying a chemical compound as either agonist or antagonist with respect to the function of protein involved in a protein-chemical compound interaction characterized by said protein-chemical compound interaction being biologically significant.
112. Method of screening chemical compounds characterized by the use of a protein involved in a biologically significant protein-chemical compound interaction as drug target.
113. Method of screening chemical compounds characterized by the use of a protein involved in a biologically significant protein-chemical compound interaction as drug target to find either agonist or antagonist with respect to the function of said protein.
114. Method of screening chemical compounds according to any of items 112 and 113, wherein affinity assay is used.
115. Method of screening chemical compounds according to any of items 112 through 114, wherein cell-based, tissue-based, organ-based, and whole animal-based systems, separately or in a combined manner, are used.
116. Method of identifying a chemical compound found by the use of the screening method claimed in any of items 112 through 115 as either of agonist or antagonist characterized by the use of an assay method wherein a functional indicator is used.
117. Method of identifying a chemical compound found by the use of the screening method claimed in any of items 112 through 115 as either of agonist or antagonist according to item 116, wherein said functional indicator is any or any combination of (a) extracellular and/or intracellular pH, (b) extracellular and/or intracellular concentrations of (b1) calcium, (b2) cyclic AMP and/or (b3) any of other biologically relevant substances, (c) optical change, (d) morphological change and (e) electrophysiological change.
118. Method of identifying a chemical compound involved in a biologically significant protein-chemical compound interaction as either of agonist or antagonist characterized by comparing the expression profile at mRNA level obtained by the use of said chemical compound with that obtained by the use of an antisense molecule corresponding to the protein involved in said protein-chemical compound interaction.
119. Method of identifying a chemical compound found by the use of the screening method claimed in any of items 112 through 115 as either of agonist or antagonist characterized by comparing the expression profile at mRNA level obtained by the use of said chemical compound with that obtained by the use of an antisense molecule corresponding to the protein involved in said protein-chemical compound interaction.
120. Use of solid support carrying a chemical compound in separation of proteins and/or portions of proteins with affinity for said chemical compound.
121. Use of solid support carrying a chemical compound according to item 120, wherein said solid support is in the form of bead and is loaded into a chromatographic column.
122. Use of solid support carrying a chemical compound according to item 120, wherein said solid support is in the form of plate.
123. Use of solid support carrying a chemical compound according to item 122, wherein said solid support is in the form of well.
124. Use of solid support carrying a chemical compound in separation of proteins or portions of said proteins with affinity for said chemical compound according to any or any combination of items 120 through 123, wherein elution of proteins or portions of said proteins with affinity for said compound is accomplished by application of a solution containing said compound in free form.
125. A multiplexed system comprising solid support with attached chemical compounds, wherein each of said chemical compounds is placed separately.
126. A multiplexed system comprising solid support with attached chemical compounds according to item 125, wherein said solid support is in the form of multiples of wells.
127. A multiplexed system comprising solid support with attached chemical compounds according to item 126, wherein a single pore is, or multiple pores are, made in each well after affinity reaction is completed.
128. A multiplexed system comprising solid support with attached chemical compounds according to item 125, wherein said solid support is in the form of a plate consisting of multiplexed mini-chromatographic columns.
129. Use of multiplexed system according to any of items 126 through 128 alone or in any combination thereof in separation of proteins or portions of proteins with affinity for said attached chemical compounds.
130. Use of solid support carrying a mixture of different chemical compounds in differential separation of proteins or portions of proteins with affinity for said chemical compounds.
131. Use of solid support carrying a mixture of different chemical compounds in differential separation of proteins or portions of proteins with affinity for said chemical compounds according to item 130, wherein differential elution of proteins or portions of proteins is accomplished by stepwise application of solutions containing said chemical compounds in free form.
132. Use of solid support carrying a mixture of different chemical compounds in differential separation of proteins or portions of proteins with affinity for said chemical compounds according to any or any combination of items 130 and 131, wherein said solid support is in the form of bead, each kind of which carries a single chemical compound, and is loaded into a chromatographic column.
133. Use of solid support carrying a mixture of different chemical compounds in differential separation of proteins or portions of proteins with affinity for said chemical compounds according to any or any combination of items 130 and 131, wherein said solid support is in the form of plate.
134. Use of solid support carrying a mixture of different chemical compounds in differential separation of proteins or portions of proteins with affinity for said chemical compounds according to any or any combination of items 130 and 131, wherein said solid support is in the form of well.
135. Use of chemical compound-attached solid support to capture cells carrying a protein or a portion of a protein on cell surface.
136. Use of chemical compound-attached solid support to capture cells carrying a protein or a portion of a protein on cell surface according to item 135, wherein said solid support is a multiplexed system.
137. Use of chemical compound-attached solid support to capture cells carrying a protein or a portion of a protein on cell surface according to any of item 135 and item 136, wherein said solid support is in the form of either bead, plate, or well.
138. Use of chemical compound-attached solid support to capture cells carrying a protein or a portion of a protein on cell surface according to any or any combination of items 135 through 137, wherein said cells have been genetically engineered to express on their surface a specific protein in an enriched quantity.
139. Use of antibody to a protein or a portion of a protein present on cell surface to liberate bound cells that carry said protein or said portion of a protein in the use of chemical compound-attached solid support to capture cells carrying said protein or said portion of a protein on cell surface claimed in any or any combination of items 135 through 138.
140. Use of sorted protein mixtures with respect to class, subcellular localization and/or function in evaluating the interaction between a protein or a portion of a protein and a chemical compound.
141. Use of sorted protein mixtures according to item 140, wherein said sorted protein mixture consists of any or any combination of secretable proteins or portions of said proteins, cell surface proteins or portions of said proteins, proteins or portions of said proteins capable of migrating into cell nucleus, GPCR proteins or portions of said proteins, phosphorylated proteins or portions of said proteins, kinases or portions of said kinases, biotinylated phosphorylated proteins or portions of said proteins, inflammatory proteins or portions of said proteins, cytokines or portions of said cytokines, and interleukins or portions of said interleukins.
142. A collection of data, database, or catalog according to item 33, wherein said extracellular virions are from baculovirus.
143. Method of evaluating the interaction between a protein or a portion of a protein and a chemical compound according to item 50, wherein said extracellular virions are from baculovirus.
144. Use of surface plasmon resonance measurement in evaluating the interaction between a protein or a portion of a protein and a chemical compound, wherein either chemical compound or protein is attached to solid support.
145. Method of evaluating the interaction between a protein or a portion of a protein and a chemical compound, wherein said method does not require chemical modification of said chemical compound.
146. Method of evaluating the interaction between a protein or a portion of a protein and a chemical compound according to item 145, wherein technology of size fractionation is used.
147. Method of evaluating the interaction between a protein or a portion of a protein and a chemical compound according to any of items 145 and 146 in the sequential steps of:
   (1)A chemical compound to be evaluated is mixed with a library containing proteins and/or portions of proteins and, after allowing some time for interaction to occur, resulting mixture is subjected to gel filtration or ultrafiltration under a condition where dissociation of said chemical compound with proteins or portions of proteins in said library is avoided.
   (2)Step (1) is repeated until most of proteins or portions of proteins in said library are separated into fractions whereby each of said fractions contains a single species of protein or a single species of portion of a protein.
   (3)Each fraction resulting from Steps (1) and (2) that contains a single species of protein or a single species of portion of a protein is then subjected to a condition that effectively liberates said chemical compound from proteins or portions of proteins in said library and is further subjected to gel filtration, ultrafiltration, or dialysis.
   (4)Each fraction resulting from Step (3) is examined for the presence or absence of said chemical compound. If present, said chemical compound is concluded to bind to said single species of protein or portion of a protein.
   (5)Sum of the amounts of said chemical compound resulting from Step
   (4) is converted to original concentration in corresponding fraction resulting from Step (3). Said original concentration and the concentration of corresponding single species of protein or portion of a protein in each of fractions resulting from Step (3) give quantitative information on the intensity of affinity of said chemical compound for said single species of protein or portion of a protein.
148. Method of evaluating the interaction between a protein or a portion of a protein and a chemical compound according to item 147, wherein said condition that effectively liberates said chemical compound from the protein is attained by the adjustment of pH, the application of high ionic strength and the use of a water-miscible organic solvent, either singly or in a combined manner.
149. Method of evaluating the interaction between a protein or a portion of a protein and a chemical compound according to item 148, wherein said water-miscible organic solvent is any or any combination of glycol, methanol, ethanol, propanol, acetonitrile, dimethyl sulfoxide, tetrahydrofuran, and trifluoroacetic acid.
150. Method of evaluating the interaction between a protein or a portion of a protein and a chemical compound according to any of items 147 through 149, wherein size exclusion chromatography including gel filtration is used in Steps (1) and/or (2) and ultrafiltration is used in Step (3).
151. Method of evaluating the interaction between a protein or a portion of a protein and a chemical compound according to any of item 146 through 150, wherein evaluation is made in mixture-versus-mixture mode.
152. Method of evaluating the interaction between a protein or a portion of a protein and a chemical compound according to item 151, wherein differential detection or quantification is employed for a group of different compounds.
153. Method of evaluating the interaction between a protein or a portion of a protein and a chemical compound characterized by the use of said protein or said portion of a protein attached to solid support.
154. Method of evaluating the interaction between a protein or a portion of a protein and a chemical compound according to item 153, wherein wells or mini-chromatographic columns with attached protein or portion of a protein after interaction is complete is subjected to steps of:
   (1)washing,
   (2)application of a compound-liberating condition, and
   (3)evaluation of liberated compound.
155. Use of determination of the change in resonant frequency of quartz oscillator or determination of the change in surface elastic wave in detecting or quantifying the interaction between a chemical compound and a protein or a portion of protein.
156. Use of determination of the change in resonant frequency of quartz oscillator or determination of the change in surface elastic wave in detecting or quantifying the interaction between a chemical compound and a protein or a portion of protein according to item 155 in any of methods, uses, and systems claimed in items 35 through 55, 71, 75, 120 through 141, 143, and 145 through 154.
157. Use of surface plasmon resonance measurement in evaluating the interaction between a protein or a portion of a protein and a chemical compound, wherein either chemical compound or protein is attached to solid support according to item 144 in any of methods, uses, and systems claimed in items 35 through 55, 71, 75, 120 through 141, 143, and 145 through 154.
158. Use of capillary electrophoresis to separate proteins or portions of proteins in evaluating the interaction between a chemical compound and a protein or a portion of a protein.
159. Use of capillary electrophoresis to separate proteins or portions of proteins in evaluating the interaction between a chemical compound and a protein or a portion of a protein according to item 158 in any of methods claimed in items 35 through 55, 71, 75, 143, and 145.
160. Use of mass analysis for detection or quantification in evaluating the interaction between a chemical compound and a protein or a portion of protein.
161. Use of mass analysis for detection or quantification in evaluating the interaction between a chemical compound and a protein or a portion of protein according to item 160 in any of methods, uses, and systems claimed in items 35 through 55, 71, 75, 120 through 141, 143 through 154, 158, and 159.
162. Method of evaluating the effect of a chemical compound on a protein-protein interaction or on a complex comprising a multitude of different proteins characterized by allowing said chemical compound to interact with a pre-formed complex or with a mixture comprising proteins that are to form said complex.
163. Method of evaluating the effect of a chemical compound on a protein-protein interaction or on a complex comprising a multitude of different proteins according to item 162 characterized by initiating the formation of said complex either by adding a component protein or by adding a reagent needed for the formation of said complex.
164. Method of evaluating the effect of a chemical compound on a protein-protein interaction or on a complex comprising a multitude of different proteins according to item 163, wherein said reagent is ATP.
165. Method of evaluating the effect of a chemical compound on a protein-protein interaction or on a complex comprising a multitude of different proteins characterized by the use of a cell.
166. Method of evaluating the effect of a chemical compound on a protein-protein interaction or on a complex comprising a multitude of different proteins characterized by the use of a cell according to item 165 characterized by transfecting a cell with a DNA sequence coding for a protein that serves as bait and, after said protein is expressed in said cell, pulling down said protein from lysate of said cell with the use of affinity chromatography for said protein.
167. Method of evaluating the effect of a chemical compound on a protein-protein interaction or on a complex comprising a multitude of different proteins characterized by the use of a cell according to item 166 characterized by transfecting said cell with a composite gene comprising a DNA sequence coding for a protein that serves as bait, a DNA sequence coding for a protein or polypeptide that serves as affinity hook and a linker DNA sequence coding for a peptide that can be cleaved by a peptidase that is specific for said peptide.
168. Method of evaluating the effect of a chemical compound on a protein-protein interaction or on a complex comprising a multitude of different proteins characterized by the use of a cell according to item 167, wherein said composite gene comprises a DNA sequence coding for a protein that serves as bait, DNA sequences coding for proteins and/or polypeptide that serve as affinity hooks and linker DNA sequences coding for peptides that can be cleaved by peptidases each of which is specific for each of said peptides.
169. Method of evaluating the effect of a chemical compound on a protein-protein interaction or on a complex comprising a multitude of different proteins according to any of items 162 through 168, wherein composition of said complex is compared in the presence and absence of said chemical compound.
170. Method of evaluating the biological significance of the effect of a chemical compound on a protein-protein interaction or on a complex comprising a multitude of different proteins characterized by comparison of composition of said complex in the presence and absence of said chemical compound.
171. Method of evaluating the biological significance of the effect of a chemical compound on a protein-protein interaction or on a complex comprising a multitude of different proteins according to item 170, wherein said comparison is performed with use of a cell.
172. Method of altering the function of a complex comprising a multitude of different proteins characterized by combinatorial use of different small molecules binding to different proteins that are constituents of said complex.
173. Therapeutic use of the method according to item 172, wherein a combination of different small molecules binding to different proteins constituting said complex is used.
174. A combination of different small molecules binding to different proteins that are constituents of a complex for therapeutic use.
175. Method of evaluating the effect of a chemical compound on a protein-protein interaction or on a complex comprising a multitude of different proteins according to any of items 162 through 169 characterized by use of any or any combination of chemical compound-attached solid support, protein-attached solid support, size fractionation, liquid chromatography, affinity chromatography, capillary electrophoresis, surface plasmon resonance measurement, determination of the change in resonant frequency of quartz oscillator, determination of the change in surface elastic wave and mass analysis.
176. Method of evaluating the biological significance of the effect of a chemical compound on a protein-protein interaction or on a complex comprising a multitude of different proteins according to any of items 170 and 171 characterized by use of any or any combination of chemical compound-attached solid support, protein-attached solid support, size fractionation, liquid chromatography, affinity chromatography, capillary electrophoresis, surface plasmon resonance measurement, determination of the change in resonant frequency of quartz oscillator, determination of the change in surface elastic wave and mass analysis.
177. Method of evaluating the interaction between a protein or a portion of a protein and a chemical compound comprising the sequential steps of:
   (1)transfecting a cell with a vector carrying a tagged gene,
   (2)allowing said cell to express corresponding protein with corresponding tag,
   (3)treating said cell with a chemical compound,
   (4)lysing said cell,
   (5)subjecting resulting cell lysate, directly or after appropriate step(s) of purification for protein fraction, to affinity separation, batch-wise or by chromatography, for the tag to obtain eluates under the condition where dissociation of said chemical compound from protein is avoided,
   (6)subjecting said eluates resulting from Step (5) to mass analysis, and
   (7)comparing resulting mass spectrum with that obtained in the absence of the treatment with said chemical compound.
178. Method of evaluating the interaction between a protein or a portion of a protein and a multitude of different chemical compounds comprising the sequential steps of:
   (1) transfecting a cell with a vector carrying a tagged gene,
   (2) allowing said cell to express corresponding protein with corresponding tag,
   (3) treating said cell with said different chemical compounds,
   (4) lysing said cell,
   (5) subjecting resulting cell lysate, directly or after appropriate step(s) of purification for protein fraction, to affinity separation, batch-wise or by chromatography, for the tag to obtain eluates under the condition where dissociation of said chemical compounds from protein is avoided,
   (6) subjecting said eluates resulting from Step (5) to mass analysis, and
   (7) comparing resulting mass spectrum with that obtained in the absence of the treatment with said chemical compounds.
179. Method of collecting data resulting from evaluation of the interaction between a protein or a portion of a protein and a chemical compound to formulate a database or a catalog characterized by collection of all or part of information on Cᵢ, identification of chemical compound, Pⱼ, identification of protein or portion of a protein, Eₖ, environment of affinity determination, Aᵢⱼₖ, determined affinity, SCᵢ, chemical structure of Cᵢ, SPⱼ, structure of Pⱼ, SCᵢₖ, structure of Cⱼ under environment k, SPⱼₖ, structure of Pⱼ under environment k, FCᵢ, function of Cᵢ, FPⱼ, function of Pⱼ, GCᵢ, how Cᵢ was gained, GPⱼ, how Pⱼ was gained, TCᵢ, target protein for Cᵢ, TPⱼ, target protein for Pⱼ and miscellaneous attributes of chemical compound and protein or a portion of protein.
180. A database or catalog formulated by the method claimed in item 179 or formulated from data obtained by the use of any or any combination of methods, uses, and systems claimed in items 35 through 69, 71 through 73, 75 through 81, 106 through 141, 143 through 172, 175 through 178.
181. A database or catalog formulated by any or any combination of:
   1. Alignment of A_{¡jk} data of proteins or portions of proteins with affinity values higher than a predetermined level for a compound Cᵢ and/or comparison of structures of those proteins or portions of proteins.
   2. Alignment of Aᵢⱼₖ data of compounds with affinity values higher than a predetermined level for a protein or a portion of protein Pⱼ and/or comparison of structures of those compounds.
   3. Clustering and alignment of Aᵢⱼₖ data with respect to compounds and proteins or portions of proteins:
      ① by ignoring whether or not each compound has been chemically modified for purpose of affinity determination.
      ② by ignoring the difference in the method of preparation (including synthesis and extraction) of the compounds.
      ③ by ignoring whether or not each of the proteins or portions of proteins has been modified post-translationally, through protein-protein interactions, or otherwise.
      ④ by ignoring the difference in the method of preparation of the proteins or portions of proteins.
      ⑤ by ignoring the difference in the environment of affinity determination.
      ⑥ according to common structures and biological functions with respect to compounds.
      ⑦ according to common structures and biological functions with respect to the proteins or portions of proteins.
      ⑧ by combining any of the above.
182. Use of concept that consensus or consensus-equivalent partial amino acid sequence and/or structure of proteins or portions of proteins can be responsible for sharing high affinities for a compound.
183. Use of concept that consensus or consensus-equivalent partial structure and/or skeleton of compounds can be responsible for sharing high affinities for a protein or a portion of a protein.
184. Method of identifying consensus or consensus-equivalent partial amino acid sequence or structure of proteins or portions of proteins that can be responsible for sharing high affinities for a compound characterized by survey of databases or catalogs claimed in any of items 180 and 181.
185. Method of identifying consensus or consensus-equivalent partial structure or skeleton of compounds that can be responsible for sharing high affinities for a protein or a portion of a protein, characterized by survey of databases or catalogs claimed in any of items 180 and 181.
186. Method of identifying consensus or consensus-equivalent partial amino acid sequence or structure of proteins or portions of proteins that is responsible for sharing high affinities for a compound according to any of items 184 and 185, wherein said partial amino acid sequence is associated with movable structure of said proteins or said portions of proteins.
187. Method of validating or discovering critical consensus or consensus-equivalent partial structure or skeleton of chemical compounds that is responsible for sharing high affinities for a protein or a portion of said protein characterized by studying changes in Aᵢⱼₖ under gradual chemical modification of the compound in question by reduction in size, substitution, or expansion in size.
188. Method of validating or discovering critical consensus or consensus-equivalent partial amino acid sequence or structure of proteins or portions of said proteins that is responsible for sharing high affinities for a compound characterized by studying changes in Aᵢⱼₖ under graded substitution of amino acid residue of said proteins or said portions of proteins.
189. Method of predicting the chemical structure of a compound that would maximize or minimize affinity and specificity for a selected target protein characterized by the use of any or any combination of methods and concepts claimed in items 182 through 188.
190. A database according to any of items 1 through 34, 70, 74, 82, 84, 104, 105, 142, 180, and 181, wherein described in tabulated format is (a) regulatory regions of genomic DNA sequence regulating the expression of said protein, and/or (b) binding sites, on genomic DNA sequence, of transcription factors that initiate the transcription of the gene encoding said protein, and/or (c) genes regulated by any of said regulatory regions, and/or (d) proteins encoded by said genes.
191. A database according to any of items 1 through 34, 70, 74, 82, 84, 104, 105, 142, 180, 181, and 189 that is further characterized by tabulated description of proteins or portions of said proteins the expression of which is affected by administration of any or any combination of chemical compounds in any or any combination of cell-free, cell-based, tissue-based, organ-based, and whole animal-based assay systems.
192. A database according to any of items 190 and 191 that is further characterized by tabulated description of SNPs located within exons of the gene encoding said protein and/or SNPs located within regulatory regions regulating the gene encoding said protein and/or SNPs located within binding sites, on genomic DNA sequence, of transcription factors that initiate the transcription of the gene encoding said protein.
193. A database according to item 192 that is further characterized by tabulated description of positions of said SNPs located within exons of the gene encoding said protein, and/or types of said SNPs located within exons of the gene encoding said protein, and/or whether or not each of said SNPs causes an alteration of amino acid residue in corresponding protein, and/or the effect of said alteration of amino acid residue on the 3-dimentional structure of said protein and/or on biological function of said protein.
194. A database according to any of items 192 and 193 that is further characterized by tabulated description of positions and/or types of SNPs located within regulatory regions regulating the gene encoding said protein and/or within binding sites, on genomic DNA sequence, of transcription factors that initiate the transcription of the gene encoding said protein.
195. A database according to any of items 1 through 34, 70, 74, 82, 84, 104, 105, 142, 180, 181, and 190 through 194 that is further characterized by addition of tabulated description of splice variant mRNAs transcribed from a gene encoding said protein or said portion of a protein.
196. A database according to item 195 that is further characterized by tabulated description of RNA sequences of said splice variant mRNAs, amino acid sequences translated from said RNA sequences, and/or 3-dimensional structures resulting from folding of said amino acid sequences.
197. A database according to any of items 1 through 34, 70, 74, 82, 84, 104, 105, 142, 180, 181, and 190 through 196, wherein pharmacological activities and/or clinical indications of the chemical compound participating in said interaction with a protein are tabulated in the form of a profile.
198. A database of profiles derived from databases according to item 197 with respect to a plurality of chemical compounds that is further characterized by tabulated description of the presence or absence of pharmacological activity and/or the degree of pharmacological activity.
199. A database according to any of items 1 through 34, 70, 74, 82, 84, 104, 105, 142, 180, 181, and 190 through 198, wherein toxicity and adverse effects of the chemical compound participating in said interaction with a protein are tabulated in the form of a profile.
200. A database of profiles derived from databases according to item 199 with respect to a plurality of chemical compounds that is further characterized by tabulated description of the presence or absence of toxicity and adverse effects and/or the degree of toxicity and adverse effects.
201. A database characterized by tabulated description of a protein-protein interaction, wherein at least one of proteins or portions of proteins participating in said interaction is capable of interacting with a chemical compound of less than 1,600, 1,000, 600, or 500 in molecular weight and/or approved for medical use.
202. A database characterized by tabulated and/or graphical description of networks of interactions among a plurality of proteins or portions of said proteins at least one of which is capable of interacting with a chemical compound of less than 1,600, 1,000, 600, or 500 in molecular weight and/or approved for medical use.
203. A user-interface that displays, in tabulated and/or graphical format, the output from any or any combination of databases according to items 1 through 34, 70, 74, 82, 84, 104, 105, 142, 180, 181, and 190 through 202.
204. Method of searching information on a chemical compound characterized by the use of any or any combination of databases and user-interface according to items 1 through 34, 70, 74, 82, 84, 104, 105, 142, 180, 181, and 190 through 203, concerning proteins or portions of proteins that interact with said chemical compound, and/or proteins or portions of proteins that are capable of interacting with other proteins or other portions of proteins, and/or proteins or portions of proteins the expression of which is affected by said chemical compound, and/or networks of interactions involving said proteins or said portions of proteins and said chemical compound, and/or information pertaining to said chemical compound and proteins or portions of proteins involved in said networks of interactions.
205. A user-interface that displays, in tabulated and/or graphical format, the output resulting from the use of the method claimed in item 204.
206. A user-interface according to item 204 that is further characterized by expressing as a connecting line a linkage between a chemical compound and a protein or a portion of a protein and as another connecting line a linkage between a protein or a portion of a protein and another protein or another portion of a protein, wherein each of the chemical compounds and proteins or portions of proteins being expressed as a node in said networks of interactions.
207. A user-interface according to any of item 205 and item 206 that is further characterized by displaying the intensity of interaction, preferably expressed as association and/or dissociation rate constant and/or equilibrium association constant, and the degree of effects of said interaction on the expression of proteins involved in said networks of interactions.
208. A user-interface according to any and any combination of items 205 through 207 that further displays, in tabulated and/or graphical format, information concerning SNPs located within exons of the gene encoding said protein and/or SNPs located within regulatory regions regulating the gene encoding said protein and/or SNPs located within binding sites, on genomic DNA sequence, of transcription factors that initiate the transcription of the gene encoding said protein.
209. A user-interface according to any and any combination of items 205 through 208 that further displays, in tabulated and/or graphical format, information concerning positions of said SNPs located within exons of the gene encoding said protein, and/or types of said SNPs located within exons of the gene encoding said protein, and/or whether or not each of said SNPs causes an alteration of amino acid residue in corresponding protein, and/or the effect of said alteration of amino acid residue on the 3-dimentional structure of said protein and/or on biological function of said protein.
210. A user-interface according to any and any combination of items 205 through 209 that further displays, in tabulated and/or graphical format, information concerning positions and/or types of SNPs located within regulatory regions regulating the gene encoding said protein and/or within binding sites, on genomic DNA sequence, of transcription factors that initiate the transcription of the gene encoding said protein.
211. Method of searching information on a protein or a portion of a protein, collectively denoted "questioned protein," characterized by the use of any or any combination of databases and user-interfaces according to items 1 through 34, 70, 74, 82, 84, 104, 105, 142, 180, 181, 190 through 203, and 205 through 210, concerning chemical compounds that interact with questioned protein, and/or other proteins or other portions of proteins that are capable of interacting with questioned protein, and/or proteins the expression of which is affected by questioned protein, and/or networks of interactions involving part or all of said proteins or said portions of proteins including questioned protein and said chemical compounds, and/or information pertaining to each of chemical compounds and proteins or portions of proteins involved in said networks.
212. A user-interface that displays, in tabulated and/or graphical format, the output resulting from the use of the method claimed in item 211.
213. Method of searching different chemical compounds with identical or similar profiles in terms of the intensity of interactions, preferably expressed as association and/or dissociation rate constant and/or equilibrium associations constant, with proteins or portions of proteins, and/or information pertaining to each of said chemical compounds by the use of any or any combination of databases and user-interfaces according to items 1 through 34, 70, 74, 82, 84, 104, 105, 142, 180, 181, 190 through 203, 205 through 210, and 212.
214. Method of searching different proteins or different portions of proteins with identical or similar profiles in terms of the intensity of interaction, preferably expressed as association and/or dissociation rate constant and/or equilibrium association constant, with chemical compounds, and/or information pertaining to each of said proteins or said portions of proteins by the use of any or any combination of databases and user-interfaces according to items 1 through 34, 70, 74, 82, 84, 104, 105, 142, 180, 181, 190 through 203, 205 through 210, and 212.
215. A user-interface that displays, in tabulated and/or graphical format, the output resulting from the use of the method claimed in item 213 and/or item 214.
216. Method of searching different chemical compounds with identical or similar profiles in terms of pharmacological activity and clinical indication and/or information pertaining to each of said chemical compounds by the use of any or any combination of databases and user-interfaces according to items 1 through 34, 70, 74, 82, 84, 104, 105, 142, 180, 181, 190 through 203, 205 through 210, 212, and 215.
217. Method of searching different chemical compounds with identical or similar profiles in terms of toxicity and adverse effect and/or information pertaining to each of said chemical compounds by the use of any or any combination of databases and user-interfaces according to items 1 through 34, 70, 74, 82, 84, 104, 105, 142, 180, 181, 190 through 203, 205 through 210, 212, and 215.
218. A user-interface that displays, in tabulated and/or graphical format, the output resulting from the use of the method claimed in item 216 and/or item 217.
219. Method of searching different chemical compounds with identical or similar profiles in terms of both pharmacological activity and toxicity, and/or information pertaining to each of said chemical compounds by the use of any or any combination of databases and user-interfaces according to items 1 through 34, 70, 74, 82, 84, 104, 105, 142, 180, 181, 190 through 203, 205 through 210, 212, 215, and 218.
220. A user-interface that displays, in tabulated and/or graphical format, the output resulting from the use of the method claimed in item 219.
221. Method of data mining to extract the relationship between (a) the interaction of a chemical compound with proteins or portions of proteins and (b) pharmacological activity, and/or toxicity, of said chemical compound, by comparing profiles, recorded in databases and user-interfaces according to any or any combination of items 1 through 34, 70, 74, 82, 84, 104, 105, 142, 180, 181, 190 through 203, 205 through 210, 212, 215, 218, and 220, of said chemical compound with respect to interaction with proteins or portions of proteins and to pharmacological activity and/or toxicity.
222. Method of data mining according to item 221, wherein data on intensities of affinity for proteins in profile of said chemical compound along with information on the function of the protein and on the availability of the protein in particular tissues and cells are used to identify a protein or proteins responsible for particular pharmacological activity and/or toxicity.
223. A user-interface that displays, in tabulated and/or graphical format, the output resulting from the use of the method claimed in any of items 221 and 222.
224. Method of constructing a tabulated database formulated by extracting commonness or similarity, termed structural category, at any level and at any aspect with the exclusion of nonspecific structural categories from the structures of a group of different chemical compounds and listing extracted structural categories for said group of chemical compounds.
225. Method of constructing a tabulated database of structural categories according to item 224, wherein each chemical compound of said group has affinity of higher than a fixed level for a protein or a portion of a protein.
226. Method of constructing a tabulated database of structural categories formulated by any combination of databases constructed by the method claimed in item 225 for a multitude of said groups.
227. A database of structural categories constructed by the use of the method claimed in any of items 224 through 226.
228. A user-interface that displays, in tabulated and/or graphical format, the output resulting from the use of the method claimed in any of items 224 through 226 and/or the use of the database claimed in item 227.
229. A user-interface that displays, in tabulated and/or graphical format, responses from the database claimed in item 227 and/or the user-interface claimed in item 228 to queries that specify protein, chemical compound, and/or structural category.
230. Method of data mining to extract the relationship in structure of (a) chemical compounds and (b) proteins or portions of proteins having affinity for each other characterized by comparing structural categories of said chemical compounds and the 1-, 2-, and 3-D structures of said proteins or portions of proteins with profiles of interactions that are recorded in databases and user-interfaces according to any or any combination of items 1 through 34, 70, 74, 82, 84, 104, 105, 142, 180, 181, 190 through 203, 205 through 210, 212, 215, 218, 220, 223, and 227 through 229.
231. Method of data mining to extract the relationship in structure of (a) a multitude of different chemical compounds and (b) a single protein or a single portion of a protein where each of (a) has affinity for (b) characterized by the use of database and user-interface claimed in any of items 227 through 229.
232. A database constructed by the use of method claimed in any of items 230 and 231.
233. A user-interface that displays, in tabulated and/or graphical format, the output resulting from the use of method claimed in any of items 230 and 231 and/or the use of database claimed in item 232.
234. Method of probing a protein with the use of a variety of chemical compounds that has affinity for said protein and characterizing said protein with structural categories that are common or similar among said chemical compounds.
235. Method of data mining to extract the relationship in structure of (a) a multitude of different proteins or different portions of proteins and (b) a single chemical compound where each of (a) has affinity for (b) characterized by the use of databases and user-interfaces according to any or any combination of items 1 through 34, 70, 74, 82, 84, 104, 105, 142, 180, 181, 190 through 203, 205 through 210, 212, 215, 218, 220, 223, 227 through 229, 232, and 233.
236. Method of data mining to extract the relationship in structure of (a) a multitude of different proteins or different portions of proteins and (b) a single chemical compound where each of (a) has affinity for (b) according to item 235 characterized by comparing amino acid sequences of said proteins and extracting partial sequences and residues that are common or similar among said proteins.
237. Method of data mining to extract the relationship in structure of (a) a multitude of different proteins or different portions of proteins and (b) a single chemical compound where each of (a) has affinity for (b) according to item 236 characterized by finding a chain comprising partial sequences and residues that are common or similar among said proteins.
238. Method of constructing a 2- or 3-demensional map of lodging sites for a chemical compound comprising said partial sequences and residues according to item 236 or said chain according to item 237, with or without identification and characterization of associated electric fields, sites of hydrogen bonding and/or van der Waals contacts, characterized by the use of crystallographic data and/or computational modeling.
239. Method of identifying an evolutionally conserved module represented by whole or part of said chain comprising common or similar partial sequences and residues found by the method claimed in item 237 as commonly participating in the interactions of proteins with a small molecule characterized by placing queries for a wide range of proteins having affinity for said compound in a single species.
240. Method of identifying an evolutionally conserved module according to item 239 characterized further by placing said queries cross-species, covering a wide range of different species.
241. Method of constructing a 2- or 3-demensional map of lodging sites for an evolutionally conserved module found by the method claimed in any of items 239 and 240, with or without identification and characterization of associated electric fields, sites of hydrogen bonding and/or van der Waals contacts, characterized by the use of crystallographic data and/or computational modeling.
242. A database constructed by the use of method claimed in any of items 234 through 241.
243. A user-interface that displays, in tabulated and/or graphical format, the output resulting from the use of method claimed in any of items 234 through 241 and/or the use of database claimed in item 242.
244. Method of data mining to extract the relationship in structure of (a) a multitude of different chemical compounds and (b) a multitude of different proteins or different portions of proteins where each of (a) has affinity for each of (b) characterized by conducting steps of:
   (1) extracting common or similar structural categories in said compounds having affinity greater than a predetermined cutoff point for each of said proteins,
   (2) preparing a table listing common or similar structural categories of said compounds associated with each of said proteins, termed profile of association, and
   (3) predicting that proteins showing the same or similar profile of association with a set of structural categories have affinity for compounds represented by said set of structural categories, and that said proteins have at least one binding site in common or a binding site similar to each other for said compounds.
245. Method of testing validity of prediction made by the use of method claimed in item 244 characterized by studying interactions between each of said proteins and another set of compounds represented by said set of structural categories.
246. Method of data mining to extract the relationship in structure of (a) a multitude of different chemical compounds and (b) a multitude of different proteins or different portions of proteins where each of (a) has affinity for each of (b) characterized by preparing a 2 x 2 table of said compounds and said proteins and marking each of intersecting boxes of compound-protein pairs showing affinity greater than a predetermined cutoff point with a sign, or by omitting preparation of said table and by conducting steps of:
   (1) extracting consensus or consensus-equivalent partial sequences from sequences of proteins showing affinity greater than said cutoff point for each of compounds,
   (2) picking up stretches of continuous amino acid codes, termed words, from consensus or consensus-equivalent partial sequences from all sequences of said proteins,
   (3) constructing another 2 x 2 table listing words picked up from said proteins against each of said compounds for which said proteins have affinity greater than said cutoff point, while retaining information on the protein origin and the location of each word in the sequence of the protein of origin, and
   (4) assigning a chain comprising words coexisting in a protein in similar locations among said proteins as being responsible for a compound-protein interaction.
247. Method of data mining to extract the relationship in structure of (a) a multitude of different chemical compounds and (b) a multitude of different proteins or different portions of proteins where each of (a) has affinity for each of (b) according to item 246, wherein assignment of a chain is performed by incomplete matching of word set.
248. Method of constructing 3-dimensional structure of chain assigned by the use of method claimed in any of items 246 and 247 characterized by searching for model proteins bearing similar chains for which crystallographic data are available and by referring to said data.
249. A database constructed by the use of method claimed in any of items 244 through 248.
250. A user-interface that displays, in tabulated and/or graphical format, the output resulting from the use of method claimed in any of items 244 through 248 and/or the use of database claimed in item 249.
251. Method of data mining to extract the relationship between (a) interactions of proteins or portions of proteins with chemical compounds and (b) interactions of said proteins or portions of proteins with other proteins or other portions of proteins characterized by comparing profiles of interactions of proteins or portions of proteins with chemical compounds and profiles of interactions of the proteins or portions of proteins with other proteins or other portions of proteins that are recorded in databases and user-interfaces according to any or any combination of items 1 through 34, 70, 74, 82, 84, 104, 105, 142, 180, 181, 190 through 203, 205 through 210, 212, 215, 218, 220, 223, 227 through 229, 232, 233, 242, 243, 249, and 250.
252. A database constructed by the use of method claimed in item 251.
253. A user-interface that displays, in tabulated and/or graphical format, the output resulting from the use of method claimed in item 251 and/or from the use of database claimed in item 252.
254. Software enabling construction of databases and user-interfaces according to any or any combination of items 1 through 34, 70, 74, 82, 84, 104, 105, 142, 180, 181, 190 through 203, 205 through 210, 212, 215, 218, 220, 223, 227 through 229, 232, 233, 242, 243, 249, 250, 252, and 253.
255. Software enabling uses of databases and user-interfaces according to any or any combination of items 1 through 34, 70, 74, 82, 84, 104, 105, 142, 180, 181, 190 through 203, 205 through 210, 212, 215, 218, 220, 223, 227 through 229, 232, 233, 242, 243, 249, 250, 252, and 253.
256. Media recording databases, user-interfaces and software according to any or any combination of items 1 through 34, 70, 74, 82, 84, 104, 105, 142, 180, 181, 190 through 203, 205 through 210, 212, 215, 218, 220, 223, 227 through 229, 232, 233, 242, 243, 249, 250, and 252 through 255.
257. Service relevant to the use databases, user-interfaces, software and media according to any or any combination of items 1 through 34, 70, 74, 82, 84, 104, 105, 142, 180, 181, 190 through 203, 205 through 210, 212, 215, 218, 220, 223, 227 through 229, 232, 233, 242, 243, 249, 250, and 252 through 256.
258. Databases, user-interfaces, methods, software, media, and services according to any of items 1 through 257, wherein said portion of protein is expressed from corresponding non-full-length cDNA molecule.
259. Databases, user-interfaces, methods, software, media, and services according to any of items 1 through 257, wherein said protein is expressed from corresponding full-length cDNA molecule and post-translationally or otherwise modified.

## Claims

1. A method of screening for an agent that has a pharmacological effect similar to a target compound (C₀) having a desired pharmacological effect, the method comprising:
(1) contacting
(i) the target compound (C₀) that is selected from a compound library and has the desired pharmacological effect with
(ii) a population of proteins in a protein library to select one or more proteins or portions of proteins (Pⱼ) that have affinity and specificity to the target compound (C₀);
(2) using one of the proteins or portions of proteins (Pⱼ) selected in step (1) as a target protein to select one or more agents that have affinity and specificity to the target protein from the compound library; and
(3) obtaining an agent that is not identical to the target compound (C₀) used in step (1) from the one or more agents selected in step (2), as a candidate compound (C₁) that has the desired pharmacological effect.

2. The method of claim 1, further comprising the following steps (1-2) between steps (1) and (2):
(1-2) specifying structure and function of the one or more proteins or portions of proteins (Pⱼ) selected in step (1).

3. The method of claim 1, further comprising the following step (4) after step (3):
(4) confirming whether the candidate compound (C₁) actually has the desired pharmacological effect.

4. The method of claim 1, wherein the protein library is a population of proteins that are expressed from full-length cDNAs.

5. The method of claim 1, wherein the compound library is a drug library.

6. The method of claim 5, wherein the drug library is a population of drugs that have been approved for medical use.

7. The method of claim 1, wherein the compound library is a population of compounds with molecular weights of less than 1,600 Daltons.

8. The method of claim 5, wherein the drug library is a population of drugs with molecular weights of less than 1,600 Daltons.
